# EUROPÄISCHE PATENTSCHRIFT

(11) **EP 0 594 019 B1**
(45) Veröffentlichungstag und Bekanntmachung des Hinweises auf die Patenterteilung: **23.02.2000**
(21) Anmeldenummer: 93116404.0
(22) Anmeldetag: 11.10.1993
(51) Int. Cl.: C07D 401/10, A61K 31/44

(54) **1-Biphenylmethyl-pyridon Derivate, deren Herstellung und deren Verwendung als Angiotensin II Antagonisten**
1-Biphenylmethyl-pyridone derivatives, their preparation and their use as angiotensin II antagonistes
Dérivés de 1-biphénylmethyl-pyridone, leur préparation et leur utilisation comme angiotensin II antagonistes

(30) Priorität: 23.10.1992 DE 4235933; 08.06.1993 DE 4319041
(43) Veröffentlichungstag der Anmeldung: 27.04.1994
(73) Patentinhaber: BAYER AG, 51368 Leverkusen (DE)
(72) Erfinder: Dressel, Jürgen, Dr., D-42477 Radevormwald (DE); Fey, Peter, Dr., D-42111 Wuppertal (DE); Hanko, Rudolf, Dr., D-40237 Düsseldorf (DE); Hübsch, Walter, Dr., D-42113 Wuppertal (DE); Krämer, Thomas, Dr., D-42111 Wuppertal (DE); Müller, Ulrich E., Dr., D-42111 Wuppertal (DE); Müller-Gliemann, Matthsias, D-42697 Solingen-Ohligs (DE); Beuck, Martin, Dr., D-40699 Erkrath (DE); Kazda, Stanislav, Prof. Dr., D-42115 Wuppertal (DE); Wohlfeil, Stefan, Dr., D-40724 Hilden (DE); Knorr, Andreas, Dr., D-40699 Erkrath (DE); Stasch, Johannes-Peter, Dr., D-42651 Solingen (DE); Zaiss, Siegfried, Dr., D-42113 Wuppertal (DE)

(56) Entgegenhaltungen:
- EP-A- 0 487 745
- EP-A- 0 500 297
- EP-A- 0 542 059

## Beschreibung

Die Erfindung betrifft trisubstituierte Biphenyle, Verfahren zu ihrer Herstellung sowie ihre Verwendung in Arzneimitteln, insbesondere als blutdrucksenkende und antiatherosklerotische Mittel.

Es ist bekannt, daß Renin, ein proteolytisches Enzym, in vivo vom Angiotensinogen das Dekapeptid Angiotensin 1 abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

In den europäischen Patentanmeldungen EP 487 745 und 500 297 werden Pyridon-substituierte Biphenyle mit blutdrucksenkenden Eigenschaften beschrieben. Die dort beschriebenen Biphenylmethylpyridon Derivate unterscheiden sich jedoch von den erfindungsgemäßen Verbindungen der Formel I durch den Substituenten R³. Während EP 487 745 einen unsubstituierten Phenylring beschreibt (R³ = Wasserstoff) beschreibt EP 500 297 dort die Substituenten R¹⁵ und R¹⁶, die jeweils Wasserstoff oder zusammen -CH=CH-CH=CH- bedeuten. Die am 15. Mai 1993 publizierte europäische Patentanmeldung 542 059 umfaßt in ihrer breiten Substituentendefinition generisch die beanspruchten Verbindungen der vorliegenden Anmeldung. Explizit werden dort jedoch nur Verbindungen beschrieben, worin R⁵ Wasserstoff bedeutet, so daß die hier beanspruchten Verbindungen als eine neue Auswahl aus dieser älteren Anmeldung anzusehen sind.

Die Erfindung betrifft nun eine Auswahl von trisubstituierten Biphenylen der allgemeinen Formel (I) 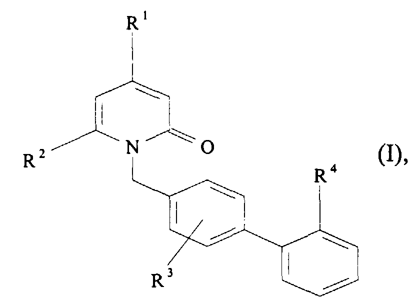 in welcher
- R¹: für einen Carboxylrest, oder für einen C₁-C₄-Alkoxycarbonylrest steht,
- R²: für geradkettiges oder verzweigtes C₁-C₅-Alkyl steht,
- R³: für Fluor, Chlor, Hydroxy, Cyano, C₁-C₃-Alkoxy, geradkettiges oder verzweigtes C₁-C₄- Alkyl, Trifluormethyl, Trifluormethoxy, Carboxamido, Carboxy, C₁-C₄-Alkoxycarbonyl oder Nitro steht, und
- R⁴: für Tetrazolyl steht, und deren Salze.

Die erfindungsgemäßen trisubstituierten Biphenyle können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt.

Physiologisch unbedenkliche Salze der trisubstituierten Biphenyle sind im allgemeinen Metall- oder Ammoniumsalze der erfindungsgemäßen Verbindungen. Besonders bevorzugt sind z.B. Lithium-, Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, entweder als Enantiomere oder als Diastereomere, existieren. Die Erfindung betrifft sowohl die Enantiomeren oder Diastereomeren als auch deren jeweiligen Mischungen. Die Racematformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962]. Außerdem ist die Bildung von atropen Isomeren möglich.

Bevorzugt sind Verbindungen der allgemeinen Formel (I), in welcher
- R¹: für Carboxy, Methoxycarbonyl oder Ethoxycarbonyl steht,
- R²: für Propyl, Butyl oder Pentyl steht,
- R³: für Fluor, Chlor, Cyano, Hydroxy, Methyl, Ethyl, Methoxy, Ethoxy, Trifluormethyl, Trifluormethoxy, Carboxamido, Carboxy, Methoxycarbonyl, Ethoxycarbonyl oder Nitro steht,
- R⁴: für Tetrazolyl steht,
und deren Salze.

Die trisubstituierten Biphenyle der allgemeinen Formel (I) werden hergestellt, indem man
[A] Pyridone der allgemeinen Formel (II) 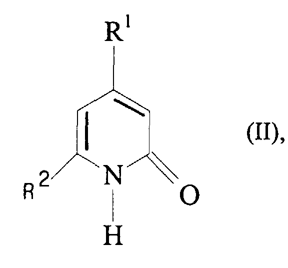 in welcher
   - R¹ und R²: die oben angegebene Bedeutung haben, mit Verbindungen der allgemeinen Formel (III) 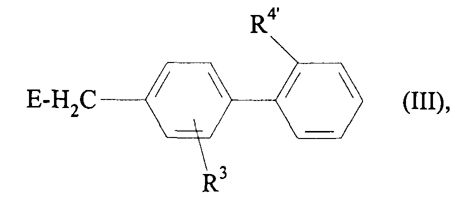 in welcher
   - R³: die oben angegebene Bedeutung hat
   - R^{4'}: für C₁-C₆-Alkoxycarbonyl oder für einen Rest der Formel 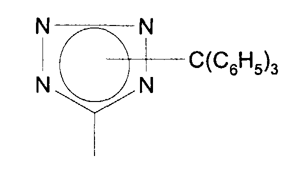 steht, und
   - E: für Chlor oder Brom steht, in inerten Lösemittel, in Anwesenheit einer Base und gegebenenfalls unter Zusatz eines Katalysators umsetzt, oder
[B] Verbindungen der allgemeinen Formel (IV) 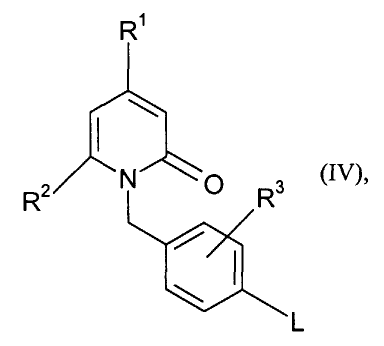 in welcher
   - R¹, R² und R³: die oben angegebene Bedeutung haben, und Methan-, Toluol-, Fluor- oder Trifluormethansulfonyloxy, vorzugsweise für Brom steht,
   mit Verbindungen der allgemeinen Formel (V) 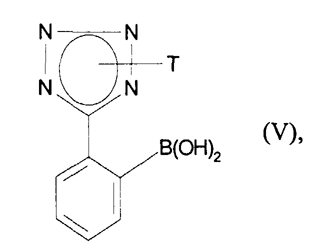 in welcher
   - T: für Wasserstoff oder für die Triphenylmethylgruppe steht,
   in inerten Lösemitteln, in Anwesenheit einer Base und metallkatalysiert, umsetzt, anschließend die Triphenylmethylgruppe mit Säuren in organischen Lösemitteln und/oder Wasser abspaltet,
im Fall der Carbonsäuren (R⁴/R¹) den entsprechendne Ester verseift und die Verbindungen gegebenenfalls mit Basen in ihre Salze überführt.

Das erfindungsgemäße Verfahren kann durch folgendes Formelschema beispielhaft erläutert werden:

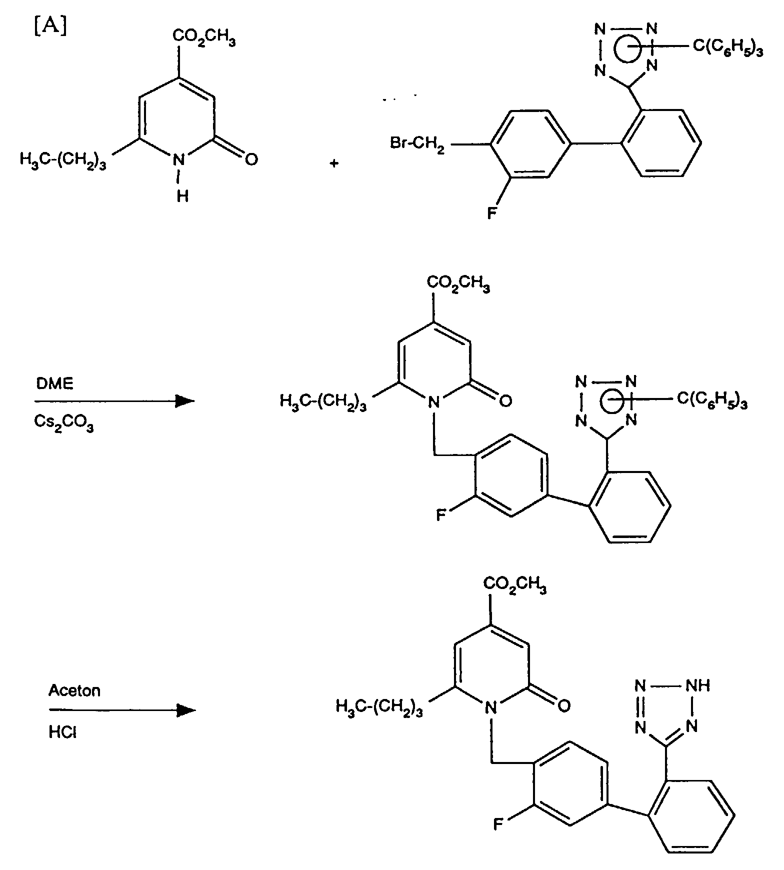

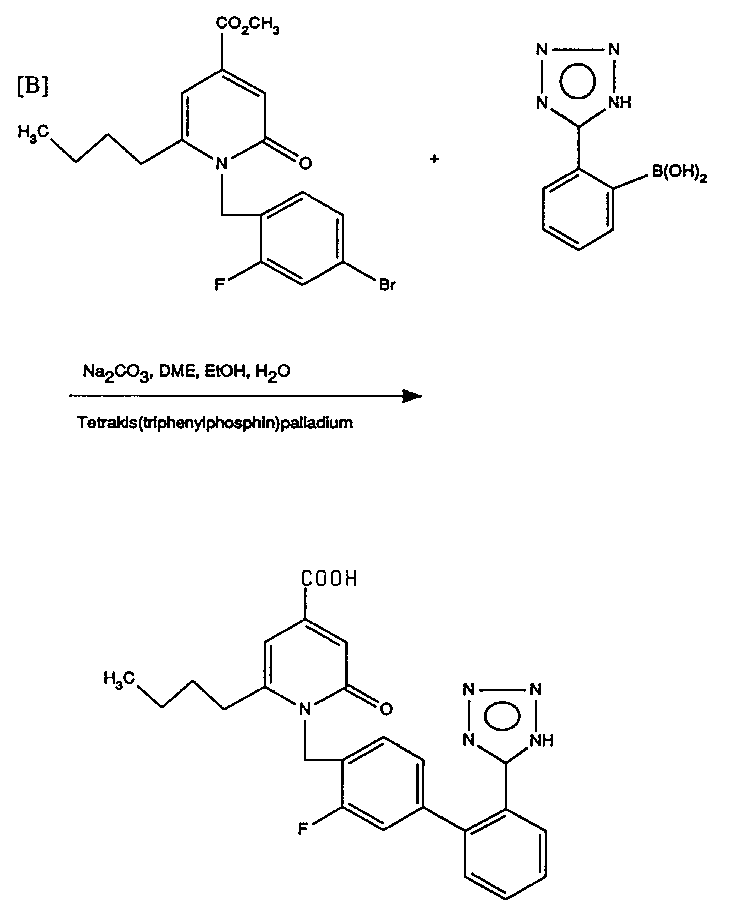

Als Lösemittel für das Verfahren [A] eignen sich übliche organische Lösemittel, die sich unter den Reaktionsbedingungen nicht verändern. Hierzu gehören bevorzugt Ether wie Diethylether, Dioxan, Tetrahydrofuran, Dimethoxyethan, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid, Hexamethylphosphorsäuretriamid, Acetonitril, Aceton oder Nitromethan. Ebenso ist es möglich. Gemische der genannten Lösemittel zu verwenden. Bevorzugt sind Tetrahydrofuran, Aceton, Dimethylformamid und Dimethoxyethan.

Als Basen für das erfindungsgemäße Verfahren können im allgemeinen anorganische oder organische Basen eingesetzt werden. Hierzu gehören vorzugsweise Alkalihydroxide wie zum Beispiel Lithiumhydroxid, Natriumhydroxid oder Kaliumhydroxid, Erdalkalihydroxide wie zum Beispiel Bariumhydroxid, Alkalicarbonate wie Natriumcarbonat oder Kaliumcarbonat, Alkali- oder Erdalkalicarbonate, wie Calcium- oder Caesiumcarbonat, oder Alkali- oder Erdalkalialkoholate oder -amide wie Natrium- oder Kaliummethanolat, Natrium- oder Kaliumethanolat oder Kalium-tert.butylat, oder Lithiumdiisopropylamid (LDA), oder organische Amine (Trialkyl(C₁-C₆)amine) wie Triethylamin, oder Heterocyclen wie 1,4-Diazabicyclo[2.2.2]-octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind Kaliumcarbonat, Natriumhydrid, Kalium-tert.-butylat oder Caesiumcarbonat.

Im allgemeinen setzt man die Base im Fall [A] in einer Menge von 0,05 mol bis 10 mol, bevorzugt von 1 mol bis 2 mol, bezogen auf 1 mol der Verbindung der Formel (III), ein.

Das erfindungsgemäße Verfahren [A] wird im allgemeinen in einem Temperaturbereich von -100°C bis +100°C, bevorzugt von 0°C bis 80°C, durchgeführt.

Die erfindungsgemäßen Verfahren werden im allgemeinen bei Normaldruck durchgeführt. Es ist aber auch möglich, die Verfahren bei Überdruck oder bei Unterdruck durchzuführen (z.B. in einem Bereich von 0,5 bis 5 bar).

Als Lösemittel für das erfindungsgemäße Verfahren [B] eignen sich übliche organische Lösemittel, die sich unter den Reaktionsbedingungen nicht verändern. Hierzu gehören bevorzugt Ether wie Diethylether, Dioxan, Tetrahydrofuran, Dimethoxyethan, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Tetrahydrofuran, Aceton, Dimethylformamid und Dimethoxyethan. Ebenso ist es möglich, in Gemischen der genannten Lösemittel mit Wasser zu arbeiten.

Das erfindungsgemäße Verfahren [B] wird im allgemeinen in einem Temperatur-bereich von -20°C bis +150°C, bevorzugt von +40°C bis +100°C durchgeführt

Als Katalysatoren eignen sich im allgemeinen Metallkomplexe des Nickeis, Palladiums oder Platins, bevorzugt Palladium(O)-Komplexe wie beispielsweise Tetrakistriphenylphosphinpalladium. Ebenso ist es möglich Phasen-Transfer-Katalysatoren, wie beispielsweise Tetra-n-butylammoniumbromid oder Kronenether einzusetzen.

Der Katalysator wird in einer Menge von 0,005 mol bis 0,2 mol, bevorzugt von 0,01 mol bis 0,05 mol, bezogen auf 1 mol der Verbindung der allgemeinen Formel (IV) eingesetzt.

Als Basen eignen sich im allgemeinen organische tert., nicht nucleophile Basen, wie beispielsweise Triethylamin oder Diisopropylethylamin oder anorganische Basen, wie Alkalicarbonate oder -hydroxide, beispielsweise Kalium-, Natrium- oder Thalliumcarbonat oder -hydroxid oder Alkoxide dieser Alkalimetalle. Bevorzugt sind Natriumcarbonat oder Kaliumcarbonat.

Im allgemeinen setzt man die Base in einer Menge von 1 mol bis 10 mol, bevorzugt von 1 mol bis 5 mol jeweils bezogen auf 1 mol der Verbindungen der Formel (IV) ein.

Gegebenenfalls werden die anorganischen Basen in wäßriger Lösung eingesetzt.

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton oder ebenfalls mit Alkoholen, oder mit einer Lösung von Chlorwasserstoff in Dioxan.

Die Abspaltung erfolgt im allgemeinen in einem Temperaturbereich von 0°C bis 150°C, vorzugsweise von 20°C bis 100°C und Normaldruck.

Als Katalysatoren eignen sich Kalium- oder Natriumiodid, bevorzugt Natriumiodid.

Als Basen für die Verseifung der Ester eignen sich die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Allcalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol, bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Verbindungen der allgemeinen Formel (II) sind teilweise bekannt und können nach bekannten Methoden hergestellt werden.

Die Verbindungen der allgemeinen Formel (III) sind teilweise bekannt oder können nach bekannten Methoden hergestellt werden.

Die Verbindung der Formel (V) ist im Fall (T = H) neu und kann hergestellt werden, indem man Phenyltetrazol zunächst unter Schutzgasatmosphäre in einem inerten Lösemittel und in Anwesenheit einer Base umsetzt und anschließend Borsäuretrimethylester zufügt und in einem letzten Schritt mit Säuren hydrolysiert.

Als Lösemittel eignen sich für das Verfahren aprotische Lösemittel wie Ether, beispielsweise Tetrahydrofuran, Diethylether, Toluol, Hexan oder Benzol. Bevorzugt ist Tetrahydrofuran.

Als Basen eignen sich prim-, sec.- und tert.Butyllithium und Phenyllithium. Bevorzugt ist n-Butyllithium.

Die Base wird in einer Menge von 2 mol bis 5 mol, bevorzugt von 2 mol bis 3 mol bezogen auf 1 mol Phenyltetrazol eingesetzt.

Als Säuren eignen sich im allgemeinen Mineralsäuren, wie beispielsweise Salzsäure, C₁-C₄-Carbonsäuren, wie beispielsweise Essigsäure oder Phosphorsäuren. Bevorzugt ist Salzsäure.

Die Säure wird im allgemeinen in einer Menge von 1 mol bis 10 mol, bevorzugt von 1 mol bis 3 mol, eingesetzt.

Das Verfahren wird im allgemeinen in einem Temperaturbereich von -70°C bis +25°C, bevorzugt von -10°C bis 0°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (IV) sind größtenteils neu und können beispielsweise hergestellt werden, indem man Verbindungen der allgemeinen Formel (VI) 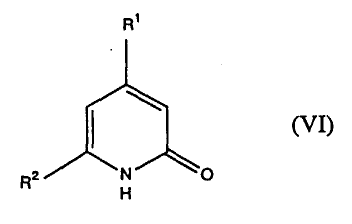 in welcher
- R¹ und R²: die oben angegebene Bedeutung haben,
mit Verbindungen der allgemeinen Formel (VII) 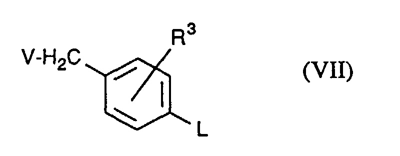 in welcher
- R³ und L: die oben angegebene Bedeutung haben
und
- V: für Halogen, vorzugsweise für Brom steht,
in inerten Lösemitteln, in Anwesenheit einer Base und/oder Katalysator umsetzt.

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid oder Dimethoxyethan, Hexamethylphosphorsäuretriamid, Acetonitril, Aceton oder Nitromethan. Ebenso ist es möglich, Gemische der genannten Lösemittel zu verwenden. Bevorzugt für das Verfahren sind Tetrahydrofuran, Aceton, Dimethylformamid, Dimethoxyethan, Alkohole wie Methanol, Ethanol oder Propanol und/oder Wasser, ToIuol und Methanol/Wasser.

Als Basen für die erfindungsgemäßen Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder Caesiumcarbonat, oder Alkali- oder Erdalkalialkoholate oder -amide wie Natrium- oder Kaliummethanolat, Natrium- oder Kaliumethanolat oder Kalium-tert.butylat, Thalliumcarbonat- oder -hydroxid, oder Lithiumdiisopropylamid (LDA), oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind für das Verfahren Kaliumcarbonat, Natriumhydrid, Kalium-tert.-butylat oder Natriumcarbonat.

Im allgemeinen setzt man die Base in einer Menge von 0,05 mol bis 10 mol, bevorzugt von 1 mol bis 2 mol jeweils bezogen auf 1 mol der Verbindungen der Formel (VII) ein.

Das erfindungsgemäße Verfahren wird im allgemeinen in einem Temperaturbereich von -100°C bis +100°C, bevorzugt von 0°C bis 80°C unter Schutzgasatmosphäre durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Katalysatoren eignen sich für das Verfahren Kalium- oder Natriumiodid, bevorzugt Natriumiodid. Ebenso ist es möglich Phasen-Transfer-Katalysatoren wie beispielsweise Tetra-n-butylammoniumbromid oder Kronenether einzusetzen.

Der Katalysator wird in einer Menge von 0,1 mol bis 10 mol, bevorzugt von 1 mol bis 2 mol, bezogen auf 1 mol der Verbindung der allgemeinen Formel (VII) eingesetzt.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar. Die erfindungsgemäßen trisubstituierten Biphenyle zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüber hinaus inhibieren sie die Proliferation von glatten Muskelzellen.

Sie können deshalb in Arzneimittel zur Behandlung der arteriellen Hypertonie und Atherosklerose eingesetzt werden. Darüber hinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

### Agonisten und ihre Standardkonzentrationen (Applikationsvolumen pro Einzelgabe = 100 µl):

| | | |
|---|---|---|
| KCl | 22,7;32,7;42,7;52,7 | mmol/l |
| 1Noradrenalin | 3x10⁻⁹;3x10⁻⁸;3x10^{-7;}3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht. Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindung mit Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), ³H-Angiotensin II (3-5 nM), Test-Pufferlösung (50 mM Tris, pH 7,2), 5 mM MgCl₂ sowie die zu untersuchenden Substanzen enthält Nach einer Inkubationszeit von 60 min bei Raumtemperatur wird die rächt gebundene Radioaktivität der Proben mittels angefeuchteter Glasfaserfilter (Whatman GF/C^{(R)}) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt.

Zur Bestimmung der IC₅₀-Werte wird die Wirkstoffkonzentration errechnet, die bei sequentieller Verdünnung des Wirkstoffes halbmaximale Hemmung der durch 10 % FCS hervorgerufene Thymidininkorporation bewirkt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hufslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel I

### N-(1-Hydroxy-2-methyl-prop-2-yl)-2-methoxy-benzoesäureamid

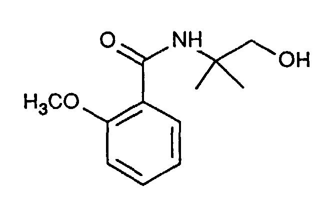

15,2 g (100 mmol) 2-Methoxy-benzoesäure werden in 300 ml Dichlormethan gelöst und bei 0°C mit 14,2 g (105 mmol) 1-Hydroxy-benzoesäuretriazol x 1 H₂O und 21,66 g (105 mmol) N,N-Dicyclohexylcarbodiimid verrührt. Die so erhaltene Suspension wird 0,5 h bei Raumtemperatur gerührt, wieder auf 0°C gekühlt und mit einer Lösung von 9,89 g (111 mmol) 1-Hydroxy-2-methyl-2-propylamin und 12,65 g (125 mmol) Triethylamin in 300 ml Dichlormethan versetzt. Nach 1 h ist die Reaktion vollständig. Die Reaktionsmischung wird mit 1 M Salzsäure und gesättigter Natriumhydrogencarbonatlösung gewaschen, über Natriumsulfat getrocknet und im Vakuum eingeengt Das Rohprodukt wird mit Petrolether verrührt, abgesaugt, mit dem Lösemittel nachgewaschen und im Hochvakuum getrocknet.

### Beispiel II

### 4,5-Dihydro-5,5-dimethyl-2-(2-methoxyphenyl)-oxazol

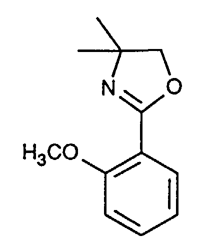

16,0 g (71,7 mmol) der Verbindung aus Beispiel I wird bei Raumtemperatur mit 17,1 ml (283,4 mmol) Thionylchlorid versetzt und 3 h gerührt. Darauf wird überschüssiges Reagenz abgedampft, der Rückstand mit 500 ml Ether ausgerührt und abgesaugt. Der Feststoff wird in Wasser gelöst, mit Ether überschichtet und die korrespondierende Base mit 2 M Natronlauge freigesetzt. Nach dreimaliger Extraktion der wäßrigen Phase mit Essigester werden die vereinigten organischen Phasen mit Natriumsulfat getrocknet, eingedampft und im Hochvakuum vom Restlösemittel befreit.

### Beispiel III

### 4,5-Dihydro-5,5-dimethyl-2-(3'-fluor-4'-methyl-biphenyl-2-yl)oxazol

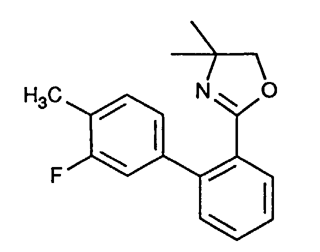

14,7 g (605,7 mol) Magnesiumspäne werden unter Argon in 50 ml Tetrahydrofuran p.a. vorgelegt und unter Rühren mit 117,7 g (623 mmol) 4-Brom-2-fluor-toluol in 500 ml Tetrahydrofuran p.a. versetzt. Bei 35 - 40°C entsteht innerhalb von 2 h eine klare Lösung. Dazu tropft man bei Raumtemperatur eine Lösung von 74,0 g (360,5 mmol) der Verbindung aus Beispiel II in 500 ml Tetrahydrofuran p.a. und rührt anfangs unter gelinder Kühlung bei ca. 25°C 16 h nach. Das Lösemittel wird abgedampft und das Rohprodukt bei 10°C in 600 ml Essigester und 800 ml gesättigter Ammoniumchloridlösung nachgewaschen, mit Natriumsulfat getrocknet und im Vakuum eingedampft. Zur Reinigung nimmt man in 600 ml Ether auf, saugt etwaigen festen Rückstand ab und zieht das Rohprodukt durch mehrfache Extraktion mit 2 M Salzsäure in die wäßrige Phase. Diese wird mit Ether überschichtet und mit Natronlauge auf pH 13 gestellt. Nach dreimaliger Extraktion mit Ether wird mit Natriumsulfat getrocknet, eingedampft und Restlösemittel im Hochvakuuum entfernt.

### Beispiel IV

### 2-(3-Fluor-4-methylphenyl)-benzonitril

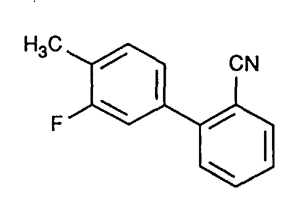

97,0 g (343 mmol) der Verbindung aus Beispiel III werden in 500 ml Pyridin vorgelegt und bei 0°C unter Rühren mit 31,3 ml (343 mmol) Phosphoroxychlorid versetzt. Das Gemisch wird langsam erwärmt und schließlich 1 h unter Rückfluß gekocht. Nach Abkühlung auf Raumtemperatur setzt man Ether und so viel 1 M Salzsäure zu, daß der pH der wäßrigen Phase bei 1,5 liegt. Die organische Phase wird noch dreimal mit 1 M Schwefelsäure gewaschen, mit Natriumsulfat getrocknet, einrotiert und im Hochvakuum vom Restlösemittel befreit.

### Beispiel V

### 5-(3'-Fluor-4'-methyl-biphen-2-yl)-1H-tetrazol

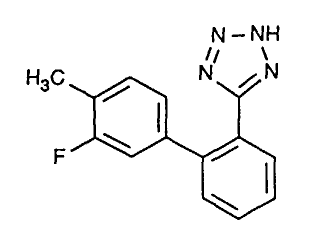

2,26 g (10,7 mmol) der Verbindung aus Beispiel IV werden mit 3,48 g (53,6 mmol) Natriumazid und 7,37 g (53,6 mmol) Triethylaminoniumchlorid in 30 ml Dimethylformamid p.a. 24 h unter Rückfluß gekocht. Nach Abkühlung verteilt man zwischen Ether und 1 M Schwefelsäure, wäscht die organische Phase mit Wasser nach, trocknet über Natriumsulfat und dampft das Lösemittel ab. Das Rohprodukt wird in Toluol ausgerührt und nach dem Absaugen im Vakuum getrocknet (1,89 g, 7,2 mmol). Die Mutterlauge wird einrotiert und erneut wie oben gereinigt (0,43 g, 1,7 mmol).

### Beispiel VI

### 5-(3-Fluor-4-methyl-biphen-2-yl)-N-triphenylmethyl-1H-tetrazol

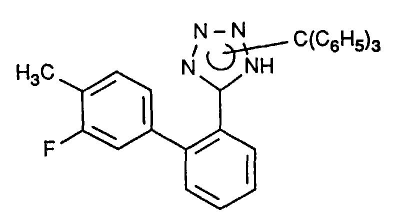

50,55 g (199,2 mmol) der Verbindung aus Beispiel V werden mit 58,58 g (210,0 mmol) Triphenylehlormethan und 33,2 ml (239,0 mmol) Triethylamin in 700 ml Dichlormethan 17 h bei Raumtemperatur gerührt. Die Reaktionsmischung wird einmal mit Wasser und einmal mit 1 M wäßriger Zitronensäure gewaschen, mit Natriumsulfat getrocknet, einrotiert und im Hochvakuum vom Restlösemittel befreit.

### Beispiel VII

### 5-(4'-Brommethyl-3'-fluor-biphen-2-yl)-N-triphenylmethyl-1H-tetrazol

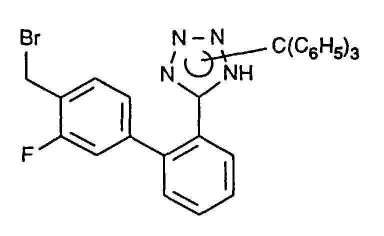

82,90 g (173,2 mmol) der Verbindung aus Beispiel VI werden mit 30,84 g (173,2 mmol) N-Bromsuccinimid und 0,87 g (5,3 mmol) Azobisisobutyronitril als Radikalstarter 6 h in 1 l Tetrachlormethan unter Rückfluß gekocht Nach dem Abkühlen wird das ausgefallene Succinimid abgesaugt und mit Tetrachlormethan gewaschen. Das Filtrat wird eingedampft und im Hochvakuum getrocknet.

### Beispiel VIII

### 6-Butyl-4-methoxycarbonyl-2-oxo-1,2-dihydropyridin

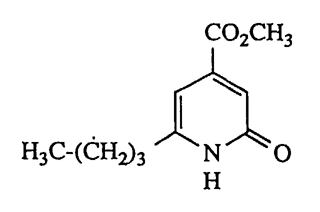

Zu einer Suspension von 29,25 g (0,15 mol) 6-Butyl-2-oxo-1,2-dihydro-isonicotinsäure in 200 ml Methanol tropft man unter Eiskühlung 12,5 ml (0,17 mol) Thionylchlorid und rührt über Nacht bei Raumtemperatur. Man engt zur Trockne ein und chromatographiert den Rückstand über 450 g Kieselgel (230-400 mesh) mit Dichlormethan → Dichlormethan/Methanol 10:1. Aus Dichlormethan, Ether, Petrolether, kristallisieren farblose Kristalle vom Schmelzpunkt 106°C.

### Beispiel IX

### 6-Butyl-4-methoxycarbonyl-2-oxo-1-{[3-fluor-2'-(N-triphenylmethyl-tetrazol-5-yl)-biphenyl-4-yl]methyl}-1,2-dihydropyridin

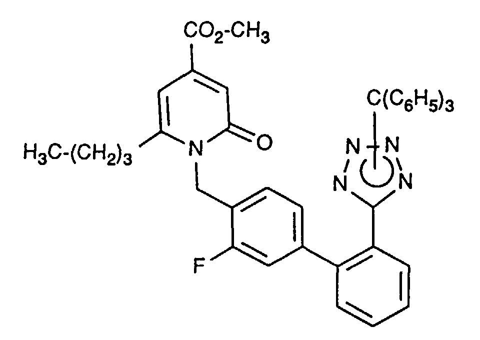

Zu einer Lösung von 31,4 g (0,15 mol) der Verbindung aus Beispiel VIII in 600 ml Dimethoxyethan gibt man 61,1 g (0,188 mol) Caesiumcarbonat, rührt 15 Minuten bei Raumtemperatur, gibt dann 104 g (0,18 mol) der Verbindung aus Beispiel VII zu, rührt über Nacht bei Raumtemperatur und kocht 3 Stunden am Rückfluß. Dann wird die Reaktionsmischung zwischen Wasser und Essigester (je 800 ml) verteilt, die organische Phase mit gesättigter Kochsalzlösung gewaschen, über Na₂SO₄ getrocknet und eingeengt. Der Rückstand wird über 2 kg Kieselgel (230 - 400 mesh) mit Petrolether / Essigester (5:1) -> (1:1) filtriert.

### Beispiel X

### 2-(Tetrazol-5-yl)phenylboronsäure

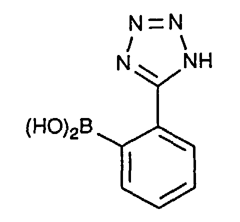

Eine Lösung von 2,9 g (20 mmol) 5-Phenyltetrazol in 50 ml THF wird bei -5°C unter Argon mir 17,6 ml (44 mmol) einer 2,5 M Lösung von n-Butyllithium in n-Hexan versetzt. Man läßt 30 min bei -5°C bis 0°C rühren und gibt bei dieser Temperatur 10 ml (88 mmol) Borsäuretrimethylester hinzu. Dann wird das Kühlbad entfernt und die Lösung bei Raumtemperatur mit 10 ml halbkonzentrierter Salzsäure versetzt. Nach 1 h wird mit 100 ml Essigester extrahiert, die organische Phase abgetrennt, und die wäßrige Phase zweimal mit je 20 ml Essigester extrahiert. Die vereinigten organischen Phasen werden über Natriumsulfat getrocknet, eingeengt und der Rückstand an Kieselgel mit Toluol / Eisessig / Methanol (38 : 0,1 : 2) gereinigt.
Ausbeute: 2,65 g (70% d.Th.)
R_{f} = 0,26 (Toluol/Methanol/Eisessig = 32:8:1)
¹³C-NMR: δ = 156,7; 137,9; 133,5; 129,8; 128,9; 127,7; 126,9 ppm.

### Beispiel XI

### 4-Brom-3-methylbenzylalkohol

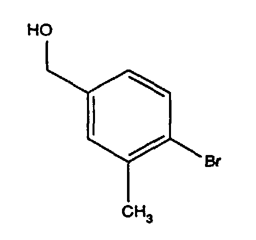

Zu einer Lösung von 10 g (46,5 mmol) 4-Brom-3-methyl-benzoesäure in 100 ml THF werden unter Argon bei 0°C 93 ml einer 1M Lösung von Boran-Tetrahydrofuran-Komplex in THF getropft. Nach Erwärmen auf 20°C wird die Reaktionsmischung 16 h bei dieser Temperatur gerührt. Anschließend wird der überschüssige Boran-Komplex durch vorsichtige Zugabe von Wasser zerstört (Ende der Wasserstoffentwicklung), zweimal mit je 250 ml Essigester extrahiert, die vereinigten organischen Phasen zweimal mit je 100 ml gesättigter Natriumhydrogencarbonat-Lösung, Wasser und gesättigter Kochsalz-Lösung gewaschen, über Natriumsulfat getrocknet und eingeengt. Die Verbindung wird ohne Reinigung weiter umgesetzt.
Ausbeute: 8 g (roh, 86 % der Theorie)
R_{f}: 0,5 (Petrolether/Essigester = 2:1)

### Beispiel XII

### 4-Brom-2-methylbenzylalkohol

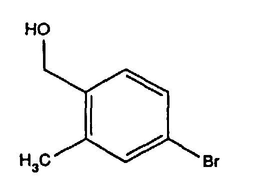

In Analogie zur Vorschrift des Beispiels XI wird die Titelverbindung aus 10 g (46,5 mmol) 4-Brom-2-methylbenzoesäure erhalten.
Ausbeute: 10 g (roh, 107 % der Theorie)
R_{f}: 0,73 (Petrolether/Essigester = 2:1)

### Beispiel XIII

### 4-Brom-3-methylbenzylbromid

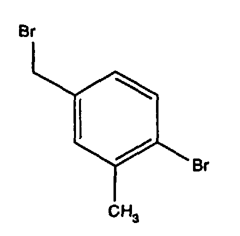

In Analogie zur Vorschrift des Beispiels XLIX wird die Titelverbindung aus 8 g (39,8 mmol) der Verbindung aus Beispiel XI erhalten.
Ausbeute: 6,4 g (61 % der Theorie)
R_{f}: 0,75 (Perrolether/Essigester = 10:1)

### Beispiel XIV

### 4-Brom-2-methylbenzylbromid

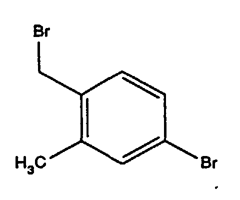

In Analogie zur Vorschrift des Beispiels XLIX wird die Titelverbindung aus 10 g (49,7 mmol) der Verbindung aus Beispiel XII erhalten.
Ausbeute: 10,9 g (83 % der Theorie)
R_{f}: 0,8 (Petrolether/Essigester = 10:1)

### Beispiel XV

### 3-Amino-6-methylbenzonitril

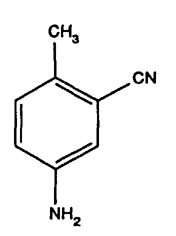

Eine Suspension von 8,11 g (50 mmol) 6-Methyl-3-nitro-benzonitril und 0,81 g 10 % Palladium auf Kohle in 50 ml Ethanol und 50 ml Essigester wird 1 h bei 2,9 bar hydriert. Der Katalysator wird abfiltriert, das Filtrat eingeengt und aus Ether/Petrolether kristallisiert.
Ausbeute: 59,6 % der Theorie
Schmelzpunkt: 88°C

### Beispiel XVI

### 3-Brom-6-methyl-benzonitril

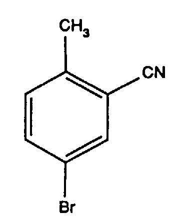

In 160 ml konz. Schwefelsäure werden 15,2 g (0,22 mol) Natriumnitrit auf 70°C erwärmt und die entstandene Lösung bei 20°C bis 40°C in eine Lösung von 26,4 g (0,2 mol) der Verbindung aus Beispiel XV in 400 ml Eisessig getropft. Zu dieser Lösung tropft man bei 10°C bis 20°C eine Lösung von 63,1 g (0,44 mol) Kupfer-(I)bromid in 400 ml konz. Bromwasserstoffsäure und rührt 0,5 h. Das Reaktionsgemisch wird in 1 l Wasser gegeben, der ausgefallene Niederschlag wird abgesaugt, mit Wasser gewaschen, in Dichlormethan suspendiert und von Unlöslichem abgesaugt. Das Filtrat wird mit gesättigter Natriumchloridlösung gewaschen, über Natriumsulfat getrocknet und zu 19,2 g der Titelverbindung eingeengt.
Ausbeute: 44,5 % der Theorie
R_{f}: 0,31 (Petrolether/Dichlormethan = 1:2)

### Beispiel XVII

### 3-Brom-6-brommethyl-benzonitril

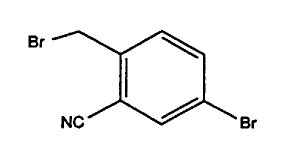

Eine Suspension aus 19,1 g (97,4 mmol) der Verbindung aus Beispiel XVI, 17,3 g (97,4 mmol) N-Bromsuccinimid und 0,2 g Azobisisobuttersäurenitril in 100 ml Tetrachlorkohlenstoff wird 1 h unter Rückfluß gerührt. Es wird filtriert, das Filtrat wird zur Trockne eingeengt und aus Methanol zu 7,7 g der Titelverbindung umkristallisiert.
Ausbeute: 28,7 % der Theorie
Schmelzpunkt: 81°C

### Beispiel XVIII

### 6-Butyl-4-methoxycarbonyl-2-oxo-1-(2-fluor-4-iod-phenylmethyl)-1,2-dihydropyridin

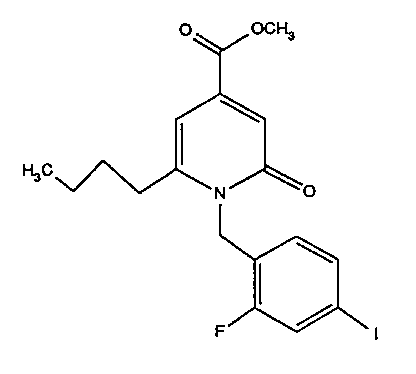

Eine Lösung von 2,09 g (10 mmol) der Verbindung aus Beispiel VIII, 3,14 g (10 mmol) 2-Fluor-4-iodbenzylbromid und 2,11 g (11 mmol) Cäsiumcarbonat in 40 ml DME werden unter Argon 16 h bei 20°C gerührt. Anschließend wird das Solvens im Vakuum entfernt, der Rückstand mit Dichlormethan/Wasser aufgenommen, die wäßrige Phase einmal mit Dichlormethan extrahiert, die vereinigten organischen Phasen über Natriumsulfat getrocknet und eingeengt. Der Rückstand wird an Kieselgel mit Petrolether/Essigester (5:1 und 3:1) gereinigt.
Ausbeute: 1,1 g (25 % der Theorie)
R_{f}: 0,44 (Petrolether/Essigester = 3:1)

In Analogie zur Vorschrift des Beispiels XVIII werden die in Tabelle I aufgeführten Verbindungen hergestellt:

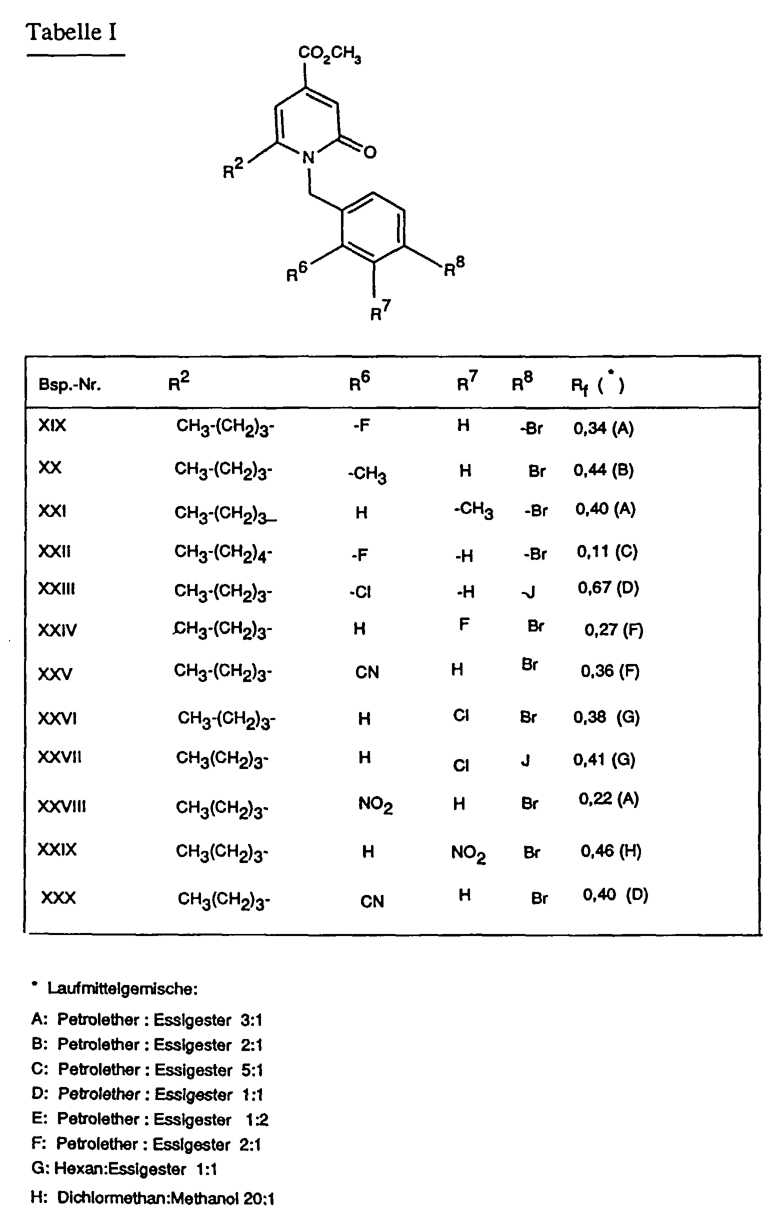

### Beispiel XXXI

### 3-Chlor-4-iod-toluol

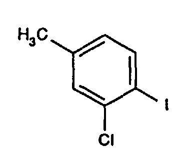

Auf 166 g Eis werden 250 ml konzentrierte Salzsäure und 75 g 4-Amino-3-chlortoluol (0,53 mol) gegeben, dann bei 0°C eine Lösung von 40,3 g Natriumnitrit (0,583 mol) in 170 ml Wasser zugetropft. Nach 15 Minuten Rühren wird die Lösung durch Glaswolle filtriert, zusammen mit einem analog mit 0,30 mol 4-Amino-3-chlor-toluol durchgeführten Ansatz wird die auf -2°C gekühlte Lösung zu einer RT-warmen Lösung von 455 g Kaliumiodid (2,74 mol) in 1 l Wasser zugetropft. Nach Rühren über Nacht wird das Reaktionsgemisch dreimal mit Ether extrahiert, die vereinten organischen Phasen werden zweimal mit verd. Natronlauge, zweimal mit verd. Natriumbisulfit-Lösung und mit Wasser gewaschen. Nach Trocknen über Natriumsulfat, Filtration und Einengen wird der Rückstand über eine Vigreux-Kolonne bei 1 mm Hg destilliert. Die Fraktion zwischen 70 und 85°C ergibt 149 g eines gelben Öls [7 % der Theorie, R_{f}= 0,57 (Hexan:Ethylacetat = 9:1)].

### Beispiel XXXII

### 3-Chlor-4-iod-benzylbromid

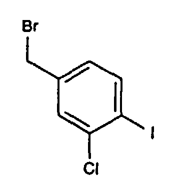

Eine Suspension von 40,4 g (160 mmol) der Verbindung aus Beispiel XXXI in 400 ml Tetrachlormethan, 31,3 g N-Bromsuccinimid (176 mmol) und 2,63 g AIBN (16 mmol) wird über Nacht zum Rückfluß erhitzt. Nach Abkühlen wird der Niederschlag abgesaugt und mit Tetrachlormethan gewaschen. Die vereinten Filtrate werden eingeengt und roh weiter umgesetzt.

### Beispiel XXXIII

### 3-Chlor-4-trifluormethylsulfonyloxy-benzoesäure-methylester

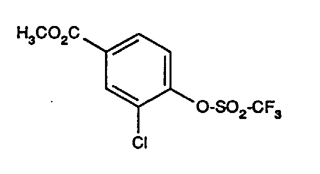

Zu einer Lösung von 5,49 g 3-Chlor-4-hydroxy-benzoesäure-methylester (29,4 mmol) in 15 ml Pyridin wird bei 0°C langsam 5,5 ml Trifluormethansulfonsäureanhydrid (33 mmol) getropft. Nach 5 min Rühren bei 0°C und 4 h bei RT wird das Reaktionsgemisch zwischen Wasser und Ether verteilt. Die organische Phase wird nacheinander mit Wasser, verd. Salzsäure, Wasser und gesättigter Kochsalzlösung gewaschen, über Natriumsulfat getrocknet, eingeengt und mit Methylenchlorid über Kieselgel chromatographiert, um 8,93 eines hellgelben dünnflüssigen Öls zu erhalten [95,2 % d. Th., R_{f} 0,63 (Hexan:Essigester = 3:1)].

### Beispiel XXXIV

### 5-(2'-Chlor-4'-methoxycarbonyl-biphen-2-yl)-2-triphenylmethyl-1H-tetrazol

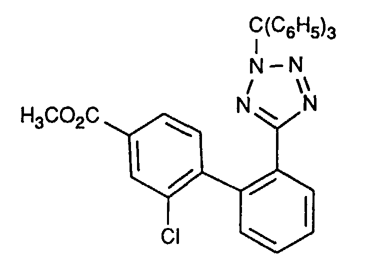

Durch eine Lösung von 1,00 g (3,14 mmol) der Verbindung aus Beispiel XXXIII in 50 ml Toluol wird Argon geleitet Nach Zugabe von 168 mg Pd (C₆H₅)₃)₄ (0,146 mmol), 6 ml Methanol, 1,63 g (3,77 mmol) 2-(N-Triphenylmethyl-tetrazol-5-yl)-phenylboronsäure und einer Lösung von 333 mg (3,14 mmol) Natriumcarbonat in 4 ml entgastem Wasser wird die Emulsion über Nacht bei 100°C gerührt. Zugabe der gleichen Menge Katalysator, gefolgt von 2,5 h Rühren bei 100°C vervollständigt die Reaktion. Das Reaktionsgemisch wird zwischen Wasser und Essigester vereilt, die organische Phase wird mit verd. Natriumcarbonat-Lösung und gesättigter Kochsalzlösung gewaschen, über Natriumsulfat getrocknet, eingeengt und über Kieselgel chromatographiert (Hexan:Essigester = 10:1), um 10,1 g eines hellgelben Feststoffs zu ergeben [57,9 % d.Th, R_{f} 0,46 (Hexan:Essigester = 3:1)].

### Beispiel XXXV

### 5-(2'-Chlor-4'-hydroxymethyl-biphen-2-yl)-triphenylmethyl-1H-tetrazol

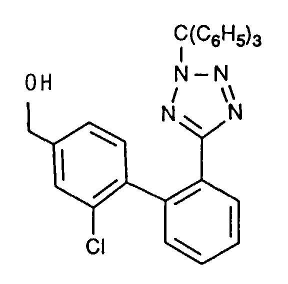

Eine Lösung von 22,0 g (39,6 mmol) der Verbindung aus Beispiel XXXIV in 180 ml THF wird mit 1,27 g Methanol (39,6 mmol) und 1,29 g Lithiumborhydrid (59,4 mmol) versetzt, dann 30 min bei RT und 1 h unter Rückfluß gerührt. Zugabe weiterer 0,63 g Methanol (0,20 mmol) und 1 h Rühren unter Rückfluß vervollständigen die Reaktion. Das Reaktionsgemisch wird eingeengt; der Rückstand wird in 200 ml Methylenchlorid aufgenommen und unter einem kräftigen Argon-Strom mit Eisbad langsam mit 100 ml N Kaliumhydrogensulfat-Lösung versetzt. Nach Trennen der Phasen wird die wäßrige Phase mit Methylenchlorid extrahiert. Die vereinten organischen Phasen werden mit gesättigter Kochsalzlösung gewaschen, über Natriumsulfat getrocknet und eingeengt, um 20,5 g weiße Kristalle zu ergeben [98,19 % d.Th.; Fp. 186-7°C (Zers.) R_{f} 0,15 (Hexan:Essigester= 3:1)].

### Beispiel XXXVI

### 5-(4'-Brommethyl-2'-chlor-biphen-2-yl)-2-triphenylmethyl-1H-tetrazol

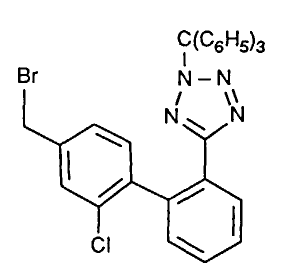

Zu einer Lösung von 11,2 g Triphenylphosphin (42,5 mmol) in 100 ml Methylenchlorid werden im Eisbad unter Argon erst 6,79 g Brom (42,5 mmol), dann 20,4 g der Verbindung aus Beispiel XXXV in 300 ml Methylenchlorid getropft. Nach 1 h Rühren bei Raumtemperatur wird das Reaktionsgemisch durch Kieselgel filtriert und mit Methylenchlorid eluiert. Einengen des Filtrats und Digerieren des Rückstands mit Hexan ergeben 15,8 g weiße Kristalle [68,9 % d.Th.; Fp. 15-60°C; R_{f} 0,40 (Hexan/Essigester = 3:1)].

### Beispiel XXXVII

### 2-Hydroxy-5-methoxycarbonyl-benzaldehydoxim

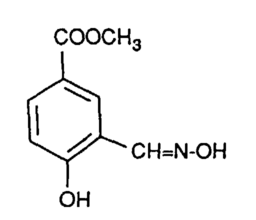

Zu einer Lösung von 80,5 g (0,45 mol) 2-Hydroxy-5-methoxycarbonylbenzaldehyd in 300 ml Methanol wird eine Lösung von 68,0 g (0,83 mol) Natriumacetat und 68,0 g (0,98 mol) Hydroxylaminhydrochlorid in 300 ml Wasser getropft und 2 h bei 25°C gerührt. Das ausgefallene Produkt wird abgesaugt, mit Wasser gewaschen und im Vakuum über Phosphorpentoxid zu 70,4 g der Titelverbindung getrocknet.
Ausbeute: 80,7 % der Theorie
Schmelzpunkt: 155°C

### Beispiel XXXVIII

### 2-Acetoxy-5-methoxycarbonyl-benzonitril

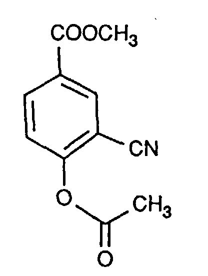

70,3 g (0,36 mol) der Verbindung aus Beispiel XXXVII werden in 0,5 l Acetanhydrid 1,5 h unter Rückfluß gerührt. Es wird zur Trockne eingeengt, in Dichlormethan gelöst, und die Titelverbindung durch Zugabe von Petrolether auskristallisiert.
Ausbeute: 89,7 % der Theorie
Schmelzpunkt: 98°C

### Beispiel XXXIX

### 2-Hydroxy-5-methoxycarbonyl-benzonitril

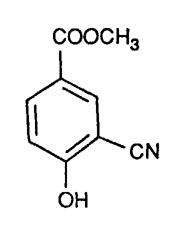

Eine Suspension von 70,6 g (0,32 mol) der Verbindung aus Beispiel XXXVIII und 3,48 g (0,06 mol) Natriummethylat in 0,5 l Methanol wird 3 h unter Rückfluß erhitzt, bei 25°C wird mit 1N Salzsäure auf pH = 6,5 eingestellt, im Vakuum eingeengt, der Rückstand in Dichlormethan aufgenommen, mit gesättigter Natriumchlorid-Lösung gewaschen, die organische Phase über Natriumsulfat getrocknet, eingeengt und aus Diethylether/Petrolethergemischen kristallisiert.
Ausbeute: 98 % der Theorie
Schmelzpunkt: 193°C

### Beispiel XL

### 3-Cyano-4-trifluormethylsulfonyloxy-benzoesäuremethylester

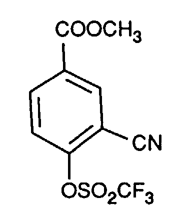

In Analogie zur Vorschrift des Beispiels XXXIX werden aus 55,8 g (0,32 mol) der Verbindung des Beispiels 98,2 g der Titelverbindung erhalten.
Ausbeute: 100 % der Theorie
R_{f} = 0,63 (Petrolether:Essigester = 2:1)

### Beispiel XLI

### 5-(2'-Cyano-4'-methoxycarbonyl-biphen-2-yl)-2-triphenyl-1H-tetrazol

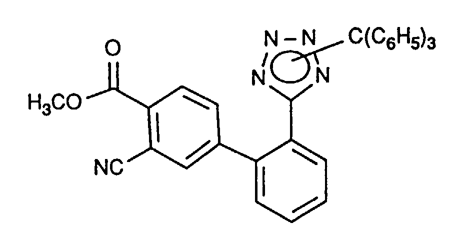

In Analogie zur Vorschrift des Beispiels XXXIV werden aus 3,0 g (10 mmol) der Verbindung des Beispiels XL 1,4 g der Titelverbindung erhalten.
Ausbeute: 26,0 % der Theorie
Schmelzpunkt: 220-240°C (Zers.)

### Beispiel XLII

### 5-(2'-Cyano-4'-hydroxymethyl-biphen-2-yl)-2-triphenylmethyl-1H-tetrazol

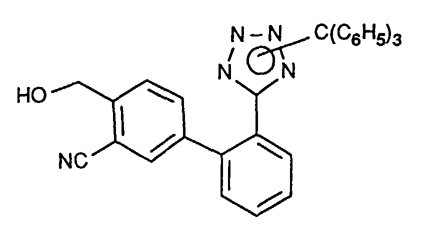

In Analogie zur Vorschrift des Beispiels XXXV werden aus 410 mg der Verbindung des Beispiels XLI 254 mg der Titelverbindung erhalten.
Ausbeute: 64,4 % der Theorie
Schmelzpunkt: 208°C

### Beispiel XLIII

### 5-(4'-Brommethyl-2'-cyano-biphen-2-yl)-2-triphenylmethyl-1H-tetrazol

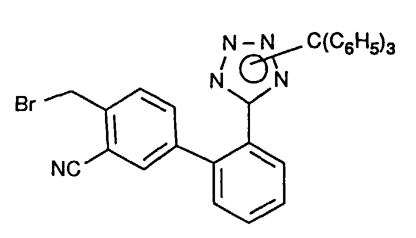

In Analogie zur Vorschrift des Beispiels XXXVI werden aus 577 mg (2,2 mmol) der Verbindung des Beispiels XLII 588 mg der Titelverbindung erhalten.
Ausbeute: 50,5 % der Theorie
Schmelzpunkt: 194°C

### Beispiel XLIV

### Trifluormethansulfonsäure-(4-formyl-3-methoxyphenyl)ester

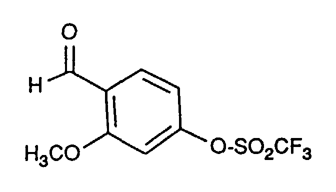

In Analogie zur Vorschrift des Beispiels XXXIII wird die Titelverbindung aus 15,2 g (0,1 mol) 2-Methoxy-4-hydroxy-benzaldehyd und 31 g (0,11 mol) Trifluormethansulfonsäureanhydrid erhalten.
Ausbeute: 15,8 g (59 % der Theorie)
R_{f}: 0,38 (Petrolether/Essigester = 20:1)

### Beispiel XLV

### Trifluormethansulfonsäure-(2-methoxy-4-methylphenyl)ester

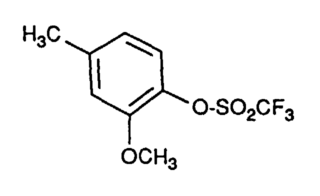

In Analogie zur Vorschrift des Beispiels XXXIII wird die Titelverbindung aus 13,8 g (0,1 mol) 2-Methoxy-4-methylphenol und 31 g (0,11 mol) Trifluormethansulfonsäureanhydrid erhalten.
Ausbeute: 26,5 g (98 % der Theorie)
R_{f}: 0,76 (Petrolether/Essigester = 3:1)

### Beispiel XLVI

### N-Triphenylmethyl-5-[2-(4'-formyl-3'-methoxybiphenyl)]tetrazol

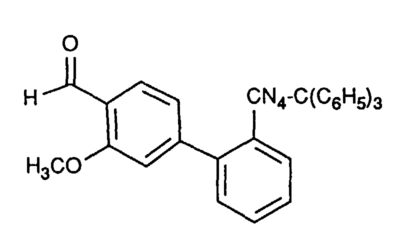

In Analogie zur Vorschrift des Beispiels XXXIV wird die Titelverbindung aus 2,46 g (10 mmol) der Verbindung aus Beispiel XLIV erhalten.
Ausbeute: 3,1 g (56 % der Theorie)
R_{f}: 0,44 (Petrolether/Essigester = 5:1)

### Beispiel XLVII

### N-Triphenylmethyl-5-[2-(2'-methoxy-4'-methylbiphenyl)]tetrazol

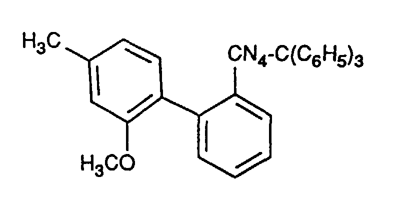

In Analogie zur Vorschrift des Beispiels XXXIV wird die Titelverbindung aus 2,0 g (7,4 mmol) der Verbindung aus Beispiel XLV erhalten.
Ausbeute: 1,75 g (47 % der Theorie)
R_{f}: 0,48 (Petrolether/Essigester = 5:1)

### Beispiel XLVIII

### N-Triphenylmethyl-5-[2-(4'-hydroxymethyl-3'-methoxybiphenyl)]tetrazol

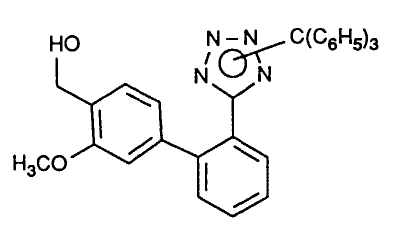

Eine Lösung von 15,9 g (30,5 mmol) der Verbindung aus Beispiel XLVI in 450 ml trockenem THF wird unter Argon bei 0°C zu 9,14 ml (9,14 mmol) einer 1M Lösung von Lithiumaluminiumhydrid in THF zugetropft. Anschließend wird das Kühlbad entfernt, 30 min bei 20°C gerührt, 50 ml Wasser und 30 ml 15 %ige Natronlauge. zugegeben und das Solvens im Vakuum entfernt. Der Rückstand wird in Dichlormethan/Wasser aufgenommen und die wäßrige Phase zweimal mit Dichlormethan extrahiert. Die vereinigten organischen Phasen werden zweimal mit gesättigter Kochsalz-Lösung gewaschen, über Natriumsulfat getrocknet und eingeengt. Der Rückstand wird an Kieselgel mit Petrolether/Essigester (10;1, 5:1, 3:1, 1:1) gereinigt.
Ausbeute: 11,5 g (72 % der Theorie)
R_{f}: 0,2 (Petrolether/Essigester = 3:1)

### Beispiel XLIX

### N-Triphenylmethyl-5-[2-(4'-brommethyl-3'-methoxybiphenyl)]tetrazol

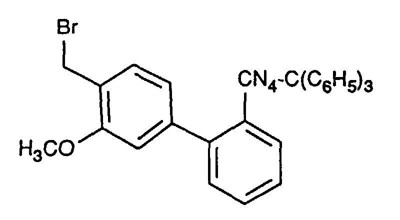

Zu einer Lösung von 11,5 g (21,8 mmol) der Verbindung aus Beispiel XLVIII in 42 ml Ether werden bei 0°C 6,2 g (23 mmol) Phosphortribromid zugetropft und 1 h bei 0°C gerührt. Die Reaktionsmischung wird auf Eiswasser gegossen, dreimal mit Essigester extrahiert, die vereinigten organischen Phasen zweimal mit gesättigter Natriumhydrogencarbonat-Lösung gewaschen, über Natriumsulfat getrocknet und eingeengt. Der Rückstand wird ohne Reinigung weiter umgesetzt.
Ausbeute: 8 g (roh, 62 % der Theorie)
R_{f}: 0,56 (Petrolether/Essigester = 5:1)

### Beispiel L

### N-Triphenylmethyl-5-[2-(4'-brommethyl-2'-methoxybiphenyl)]tetrazol

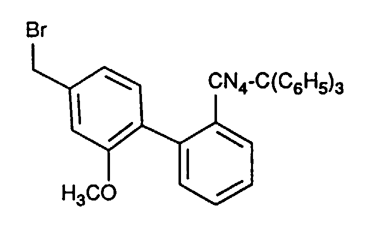

In Analogie zur Vorschrift des Beispiels VII wird die Titelverbindung aus 7,73 g (15,2 mmol) der Verbindung aus Beispiel XLVII erhalten.
Ausbeute: 6,57 g (74 % der Theorie)
R_{f}: 0,41 (Petrolether/Essigester = 10:1)

### Beispiel LI

### 6-Butyl-4-methoxycarbonyl-2-oxo-1-{[2-chlor-2'(N-triphenylmethyl-tetrazol-5-yl)-biphenyl-4-yl]-methyl}1,2-dihydropyridin

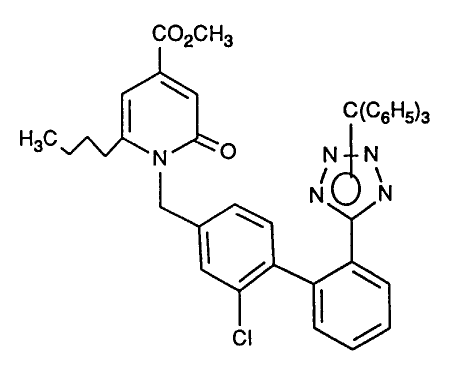

Analog zu Beispiel IX werden aus 6,11 g (29,2 mmol) der Verbindung aus Beispiel VIII und 15,7 g (26,5 mmol) der Verbindung aus Beispiel XXXVI 6,60 g der Titelverbindung erhalten [34,6 % der Theorie, R_{f} 0,35 (Hexan:Essigester = 3:1)].

In Analogie zu Vorschrift des Beispiels IX werden die in Tabelle II aufgeführten Verbindungen hergestellt:

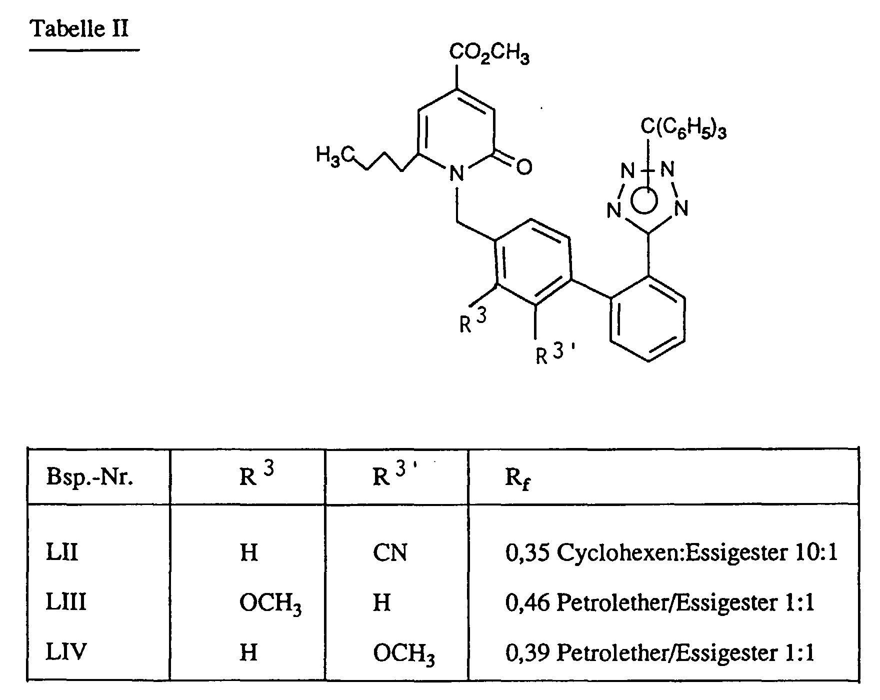

### Beispiel LV

### 6-Butyl-4-methoxycarbonyl-2-oxo-1-{[3-chlor-2'-(N-triphenylmethyl-tetrazol-5-yl)-biphenyl-4-yl]methyl}-1,2-dihydropyridin

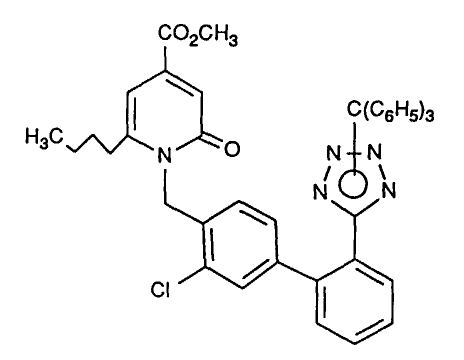

Durch eine Suspension von 5,3 g (12 mmol) der Verbindung aus Beispiel XXIII 5,0 g (12 mmol) 2-(N-Triphenylmethyl-tetrazol-5-yl)-phenylboronsäure und 1,22 g (11,5 mmol) Natriumcarbonat in 14 ml Wasser, 14 ml Methanol und 110 ml Toluol wird Argon geleitet, und danach 0,70 g (0,61 mmol) Tetrakistriphenyl-phosphinpalladium (0) zugegeben. Die Reaktionsmischung wird über Nacht auf 90°C erhitzt, bei Raumtemperatur mit Wasser verdünnt und mit Essigester extrahiert. Das Waschen der organischen Phase erfolgt mit gesättigter Kochsalzlösung, das Trocknen über Natriumsulfat. Einengen und Chromatographie über Kieselgel (Essigester:Petrolether = 1:3) ergeben 3,44 g der Titelverbindung (41 % der Theorie; R_{f} 0,23 (Essigester:Petrolether = 1:3).

In Analogie zur Vorschrift des LV werden die in Tabelle III aufgeführten Beispiele hergestellt:

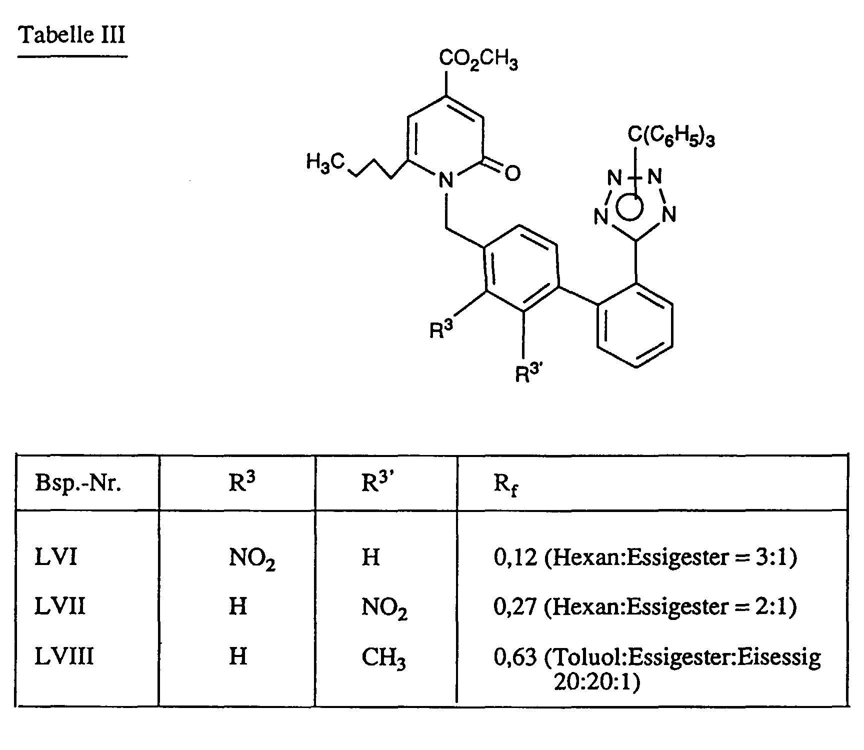

### Herstellungsbeispiele

### Beispiel 1

### 6-Butyl-4-methoxycarbonyl-2-oxo-1-[(3-fluor-2'-tetrazol-5-yl-biphenyl-4-yl)-methyl]-1,2-dihydropyridin

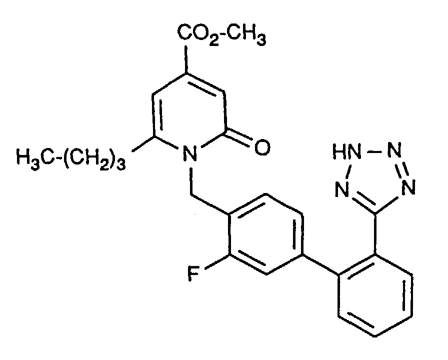

Eine Lösung von 3,0 g (4,2 mmol) der Verbindung aus Beispiel IX in 40 ml Aceton wird mit 0,4 ml 37%iger Salzsäure 30 min bei Raumtemperatur gerührt und dann ca. 1 min auf dem Wasserbad erwärmt. Nach Zugabe weiterer 0,4 mol 37%iger Salzsäure wird der Prozeß wiederholt Man engt zur Trockene ein und chromatographiert den Rückstand an 90 g Kieselgel (230-400 mesh mit Dichlormethan : Methanol 50:1 -> 20:1).
Ausbeute: 1,6g (81% der Theorie)
R_{f} = 0,49 (Toluol:Essigester:Eisessig 10:30:1)

### Beispiel 2

### 6-Butyl-4-methoxycarbonyl-2-oxo-1-{[3-methoxy-2'-(tetrazol-5-yl)-biphenyl-4-yl]-methyl}-1,2-dihydropyridin

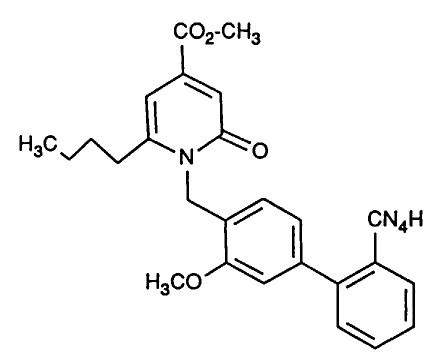

In Analogie zur Vorschrift des Beispiels 1 wird die Titelverbindung aus 435 mg (0,61 mmol) der Verbindung aus Beispiel LIII erhalten.
Ausbeute: 162 mg (56 % der Theorie)
R_{f}: 0,33 (Toluol/Essigester/Eisessig = 20:20:1)

### Beispiel 3

### 6-Butyl-4-methoxycarbonyl-2-oxo-1-{[2-methoxy-2'-(tetrazol-5-yl)-biphenyl-4-yl]-methyl}-1,2-dihydropyridin

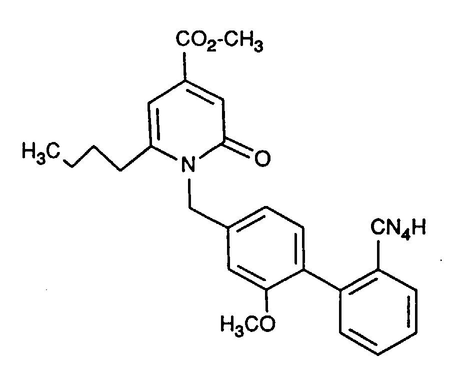

In Analogie zur Vorschrift des Beispiels 1 wird die Titelverbindung aus 740 mg (1,03 mmol) der Verbindung aus Beispiel LIV erhalten.
Ausbeute: 455 mg (93 % der Theorie)
R_{f}: 0,28(Toluol/Essigester/Eisessig = 20:20:1)

### Beispiel 4

### 6-Butyl-4-methoxycarbonyl-2-oxo-1-{[2-methyl-2'-(tetrazol-5-yl)-biphenyl-4-yl]-methyl}-1,2-dihydropyridin

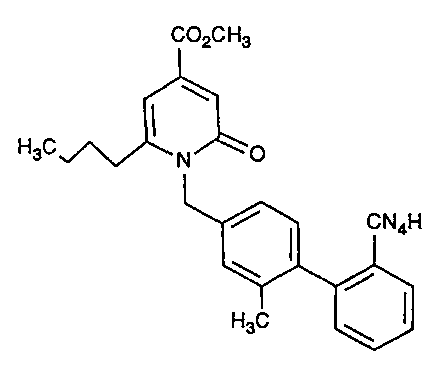

In Analogie zur Vorschrift des Beispiels 1 wird die Titelverbindung aus 1,0 g (1,42 mmol) der Verbindung aus Beispiel LVIII erhalten.
Ausbeute: 513 mg (79 % der Theorie)
R_{f}: 0,11(Toluol/Essigester/Eisessig = 30:20:1)

### Beispiel 5

### 6-Butyl-4-methoxycarbonyl-2-oxo-1-{[2-cyano-2'-(tetrazol-5-yl)-biphenyl-4-yl]-methyl}-1,2-dihydropyridin

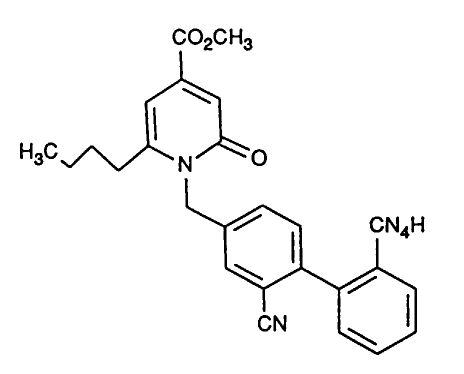

In Analogie zur Vorschrift des Beispiels 1 wird die Titelverbindung aus 1,0 g (1,13 mmol) der Verbindung aus Beispiel LII erhalten.
Ausbeute: 679 mg (91,1 % der Theorie)
Schmelzpunkt: 210-215°C (Zers.)

### Beispiel 6

### 6-Butyl-4-methoxycarbonyl-2-oxo-1-{[3-chlor-2'-(tetrazol-5-yl)-biphenyl-4-yl]-methyl}-1,2-dihydropyridin

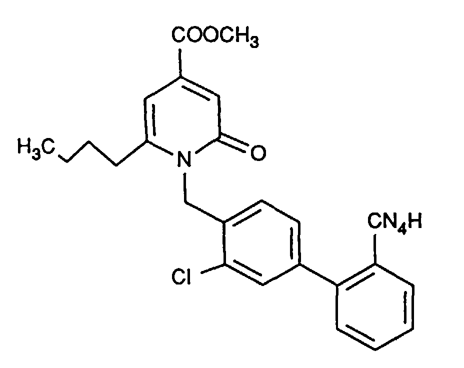

In Analogie zur Vorschrift des Beispiels 1 wird die Titelverbindung aus 3,4 g (4,7 mmol) der Verbindung des Beispiels LV erhalten.
Ausbeute: 1,7 g (75 % der Theorie)
R_{f}: 0,25 (Dichlormethan:Methanol 10:1)

### Beispiel 7

### 6-Butyl-4-methoxycarbonyl-2-oxo-1-{[2-chlor-2'-(tetrazol-5-yl)-biphenyl-4-yl]-methyl}-1,2-dihydropyridin

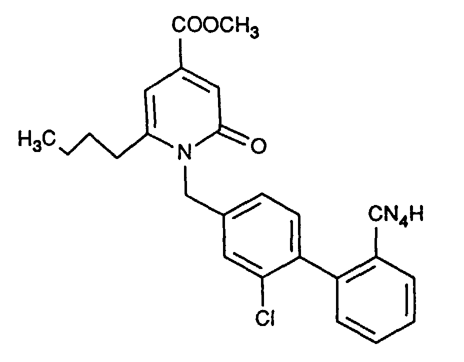

In Analogie zur Vorschrift des Beispiels 1 wird die Titelverbindung aus 394 mg (0,55 mmol) der Verbindung des Beispiels LI erhalten.
Ausbeute: 115 mg (44 % der Theorie)
R_{f}: 0,29(Dichlormethan:Methanol 20:1)

### Beispiel 8

### 6-Butyl-4-methoxycarbonyl-2-oxo-1-{[3-nitro-2'-(tetrazol-5-yl)-biphenyl-4-yl]-methyl}-1,2-dihydropyridin

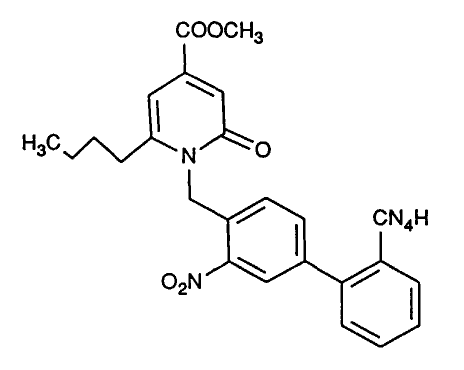

In Analogie zur Vorschrift des Beispiels 1 wird die Titelverbindung aus 390 mg (0,53 mmol) der Verbindung des Beispiels LVI erhalten.
Ausbeute: 135 mg (52 % der Theorie)
R_{f}: 0,28(Dichlormethan:Methanol 10:1)

### Beispiel 9

### 6-Butyl-4-methoxycarbonyl-2-oxo-1-{[2-nitro-2'-(tetrazol-5-yl)-biphenyl-4-yl]-methyl}-1,2-dihydropyridin

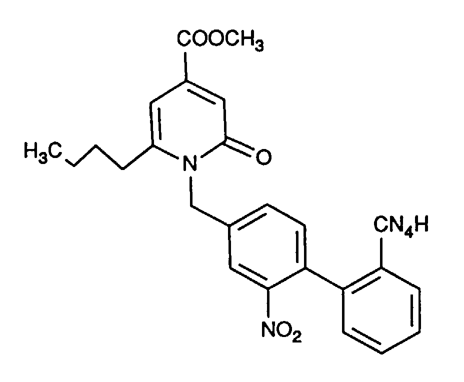

In Analogie zur Vorschrift des Beispiels 1 wird die Titelverbindung aus 790 mg (1,1 mmol) der Verbindung des Beispiels LVII erhalten.
Ausbeute: 296 mg (56 % der Theorie)
R_{f}: 0,29(Dichlormethan:Methanol 10:1)

Die in der folgenden Tabelle 1 aufgeführten Verbindungen werden analog zum Beispiel 1 hergestellt:

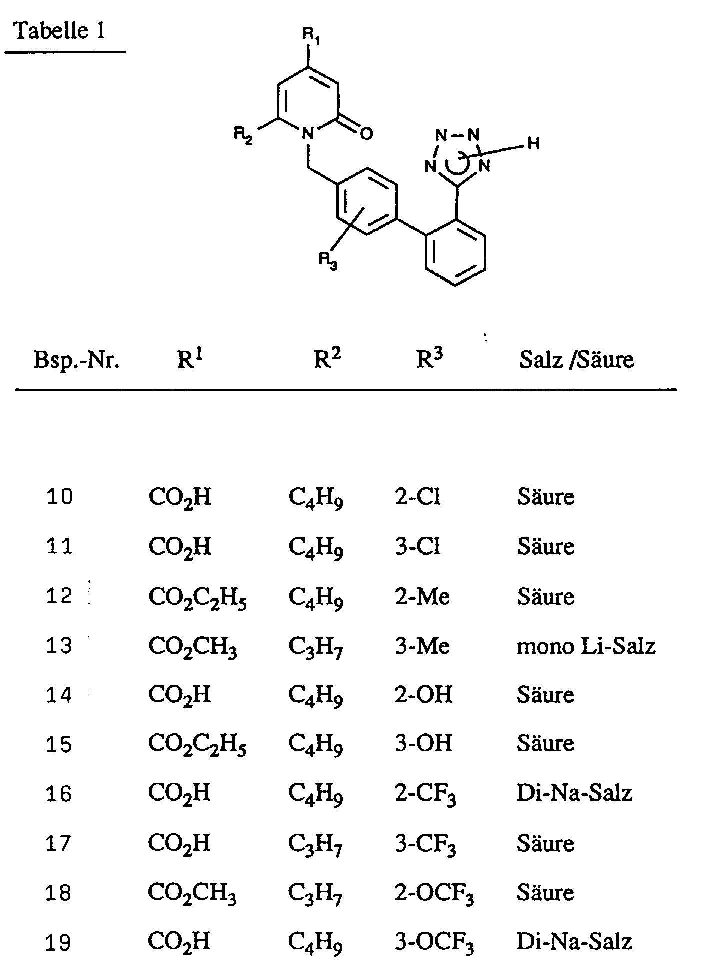

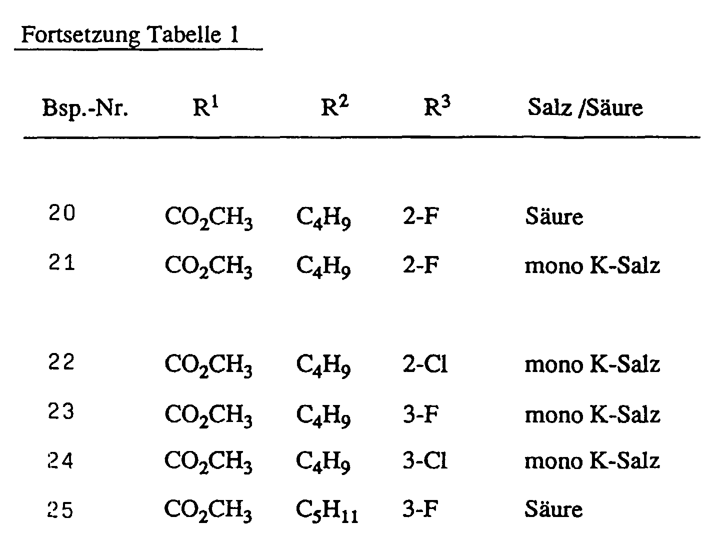

### Beispiel 26

### 6-Butyl-4-carboxy-2-oxo-1-[(3'-fluor-2'-(tetrazol-5-yl-biphenyl-4-yl)-methyl]-1,2-dihydropyridin

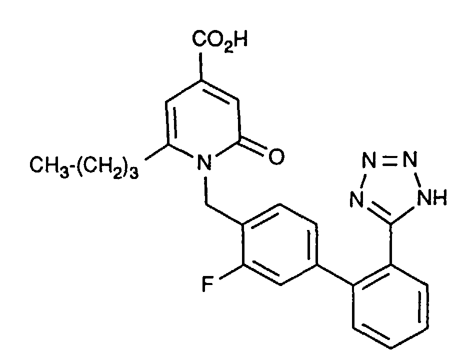

Eine Lösung von 1 g (2,25 mmol) der Verbindung aus Beispiel XVIII in 20 ml DME wird sukzessive mit 260 mg (10 mol %) Tetrakistriphenylphosphinpalladium (0), 6,75 ml (13,5 mmol) 2M Natriumcarbonatlösung, 513 mg (2,7 mmol) der Verbindung des Beispiels X und 1,5 ml Ethanol versetzt und 16 h unter Rückfluß erhitzt. Nach Abkühlen wird die Reaktionsmischung über Kieselgur abgesaugt, mit Methanol nachgewaschen, das Solvens entfernt und an Kieselgel mit Toluol/Essigester/Eisessig (35:5:1 und 30:10:1) gereinigt.
Ausbeute: 219 mg (22 % der Theorie)
R_{f}: 0,16(Toluol/Essigester/Eisessig = 10:30:1)

In Analogie zur Vorschrift des Beispiels 26 werden die in Tabelle 2 aufgeführten Verbindungen hergestellt:

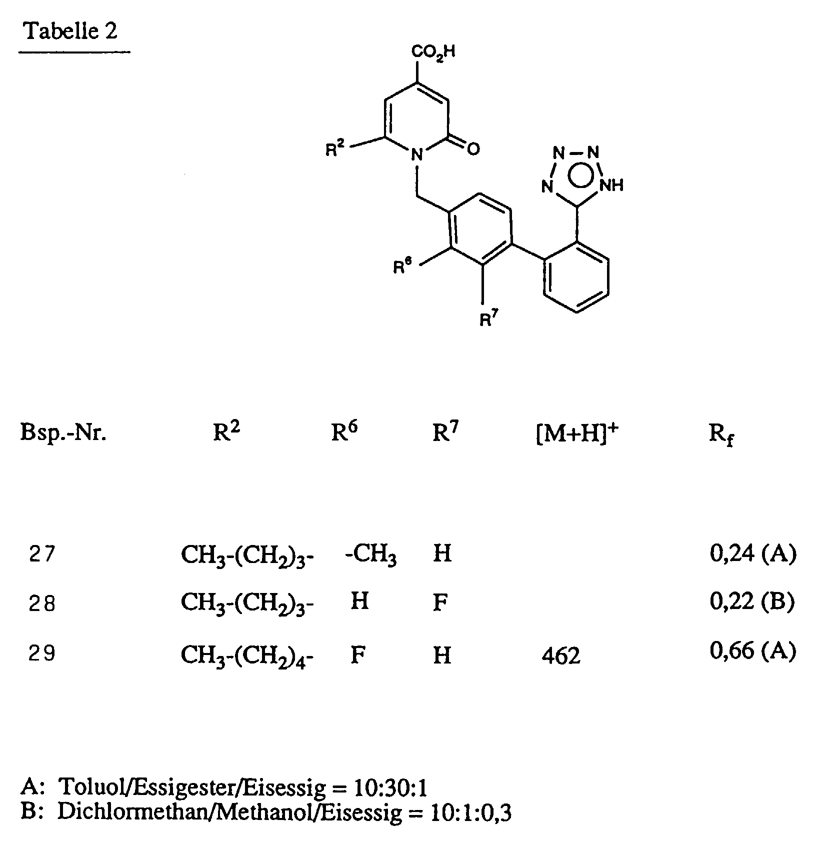

### Beispiel 30

### 6-Butyl-4-methoxycarbonyl-2-oxo-1-[(2-methoxycarbonyl-2'-tetrazol-5-yl-biphenyl -4-yl)-methyl]-1,2-dihydropyridin

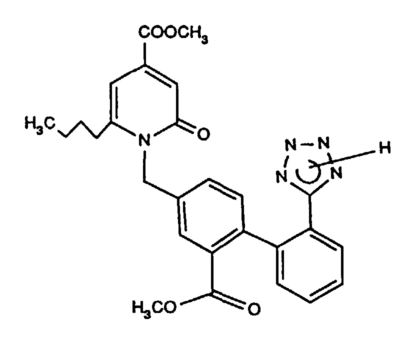

Zu einer Lösung von 166 mg (0,35 mmol) der Verbindung aus Beispiel 50 in 5 ml Methanol werden 204 ml Thionylchlorid gegeben und 3 h unter Rückfluß erhitzt. Es wird zur Trockne eingeengt und über Kieselgel mit Dichlormethan:Methanol (20:1) zu 131 mg der Titelverbindung chromatographiert.
Ausbeute: 74,6 % der Theorie
R_{f}: 0,54 (Dichlormethan:Methanol 5:1)

In Analogie zur Vorschrift des Beispiels 30 wurden die in der Tabelle 3 aufgeführten Verbindungen hergestellt:

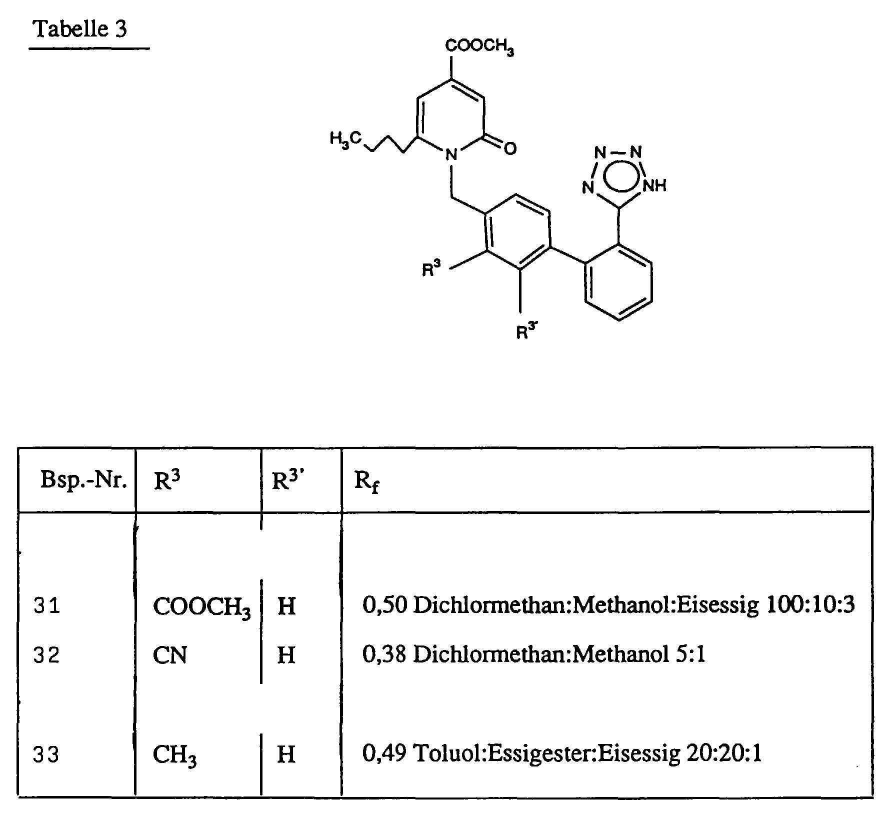

### Beispiel 34

### 6-Butyl-4-methoxycarbonyl-2-oxo-1-[(2-aminocarbonyl-2'-tetrazol-5-yl-biphenyl -4-yl)-methyl]-1,2-dihydropyridin-Kaliumsalz

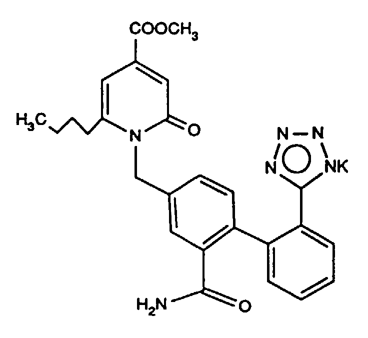

Eine Lösung von 400 mg (0,82 mmol) der Verbindung aus Beispiel 41 wird mit 1644 ml (0,82 mmol) einer 0,5 N Kaliumhydrocarbonatlösung gerührt. Es wird mit 10 ml Wasser verdünnt, das Tetrahydrofuran wird im Vakuum abdestilliert, und die wässrige Produktlösung zu 399 mg der Titelverbindung gefriergetrocknet.
Ausbeute: 92,5 % der Theorie
MS(FAB) 487 (M + H) 525 (M + K + H)

In Analogie zur Vorschrift des Beispiels 34 werden die in Tabelle 4 aufgeführten Verbindungen hergestellt:

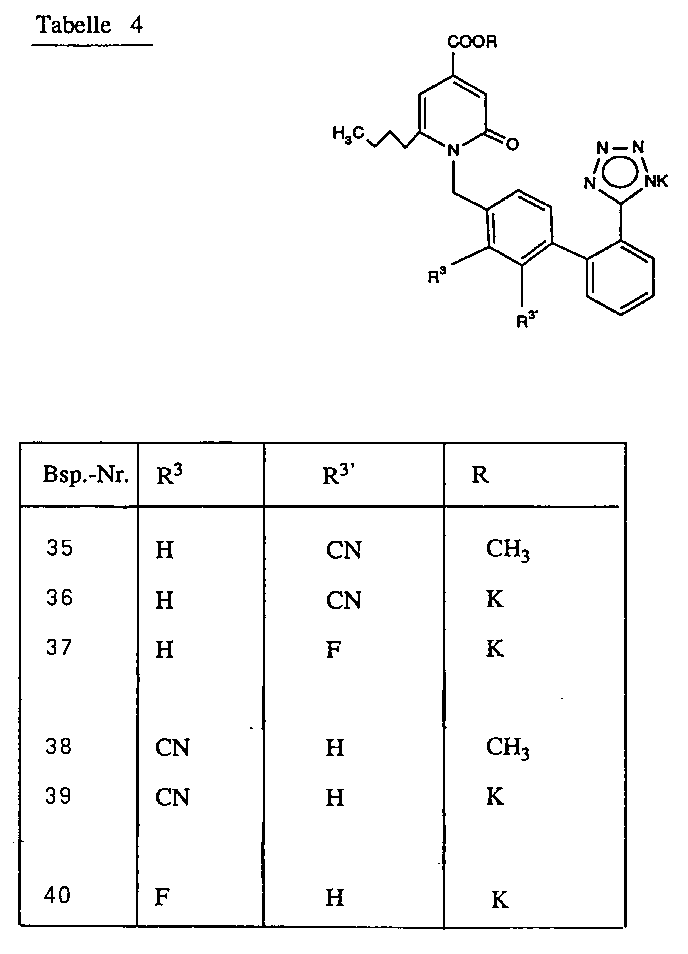

### Beispiel 41

### 6-Butyl-4-methoxycarbonyl-2-oxo-1-[(2-aminocarbonyl-2'-tetrazol-5-yl-biphenyl -4-yl)-methyl]-1,2-dihydropyridin

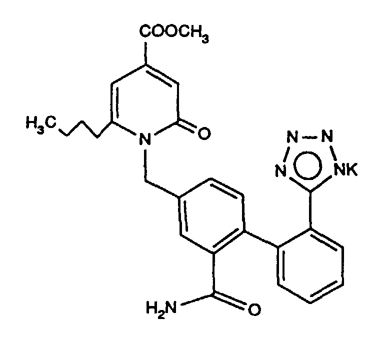

Eine Suspension aus 708 mg (1,5 mmol) der Verbindung aus Beispiel 5 in 20 ml Methanol wird mit Chlorwasserstoffgas gesättigt, und die entstandene klare Lösung 48 h bei 20°C gerührt. Es wird zur Trockne eingeengt und an Kieselgel mit Essigester:Methanol:Wassergemischen 20:1:0→100:15:5 zu 586 mg der Titelverbindung chromatographiert.
Ausbeute: 79,7 % der Theorie
R_{f} = 0,2 (Essigester:Methanol:Wasser 100:15:5)

### Beispiel 42

### 6-Butyl-4-carboxy-2-oxo-1-[(2-aminocarbonyl-2'-tetrazol-5-yl-biphenyl-4-yl)-methyl]-1,2-dihydropyridin-di-Kaliumsalz

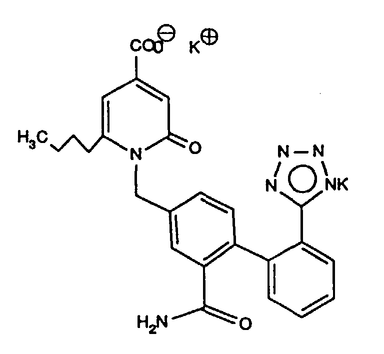

Eine Lösung von 100 mg (0,2 mmol) der Verbindung aus Beispiel 41 in 7 ml Tetrahydrofuran und 421 ml (0,421 mmol) einer 1 N-Kaliumhydroxidlösung werden 4 h bei 20°C gerührt, anschließend mit 10 ml Wasser verdünnt, das Tetrahydrofuran im Vakuum abdestilliert, und die wässrige Lösung zu 103 mg der Titelverbindung gefriergetrocknet.
Ausbeute: 91,1 % der Theorie
R_{f}: 0,15 (Dichlormethan:Methanol:Essigsäure 100:10:3)

### Beispiel 43

### 6-Butyl-4-carboxy-2-oxo-1-[(2-carboxy-2'-tetrazol-5-yl-biphenyl-4-yl)-methyl]-1,2-dihydropyridin

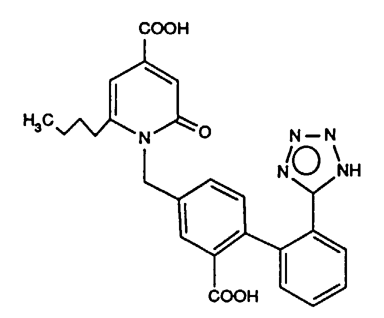

Eine Lösung von 708 mg (1,5 mmol) der Verbindung aus Beispiel 35 in 3 ml 5 N-Natronlauge wird 2 h unter Rückfluß gerührt, mit 200 ml Wasser und 10 ml Essigester bei 20°C verdünnt und mit Salzsäure auf pH = 1 gestellt. Die organische Phase wird abgetrennt und die wässrige Phase wird über Natriumsulfat getrocknet, eingeengt und an Kieselgel mit Dichlormethan:Methanol:Essigsäure-Gemischen 100:5:0,5→100:15:5 zu 204 mg der Titelverbindung chromatographiert.
Ausbeute: 37,4 % der Theorie
R_{f}: 0,10 (Dichlormethan:Methanol:Essigsäure = 100:10:5)

### Beispiel 44

### 6-Butyl-4-carboxy-2-oxo-1-[(2-aminocarbonyl-2'-tetrazol-5-yl-biphenyl -4-yl)-methyl]-1,2-dihydropyridin

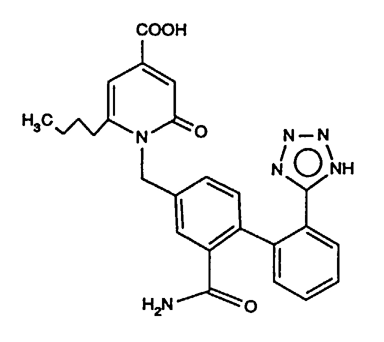

eluiert.

### Beispiel 45

### 6-Butyl-4-carboxy-2-oxo-1-{[3-methoxy-2'-(tetrazol-5-yl)-biphenyl-4-yl]-methyl}-1,2-dihydropyridin

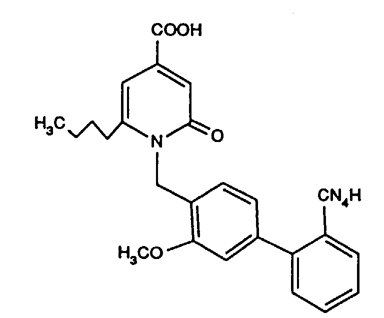

In Analogie zur Vorschrift des Beispiels 43 wird die Titelverbindung aus 135 mg (0,28 mmol) der Verbindung aus Beispiel 2 erhalten.
Ausbeute: 59 mg (45 % der Theorie)
R_{f}: 0,17 (Toluol/Essigester/Eisessig = 20:20:1)

### Beispiel 46

### 6-Butyl-4-carboxy-2-oxo-1-{[2-methoxy-2'-(tetrazol-5-yl)-biphenyl-4-yl]-methyl}-1,2-dihydropyridin

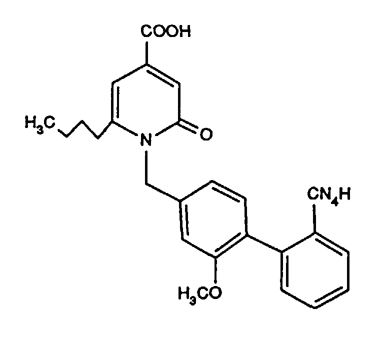

In Analogie zur Vorschrift des Beispiels 43 wird die Titelverbindung aus 393 mg (0,83 mmol) der Verbindung aus Beispiel 3 erhalten.
Ausbeute: 363 mg (95 % der Theorie)
R_{f}: 0,11 (Toluol/Essigester/Eisessig = 10:30:1)

### Beispiel 47

### 6-Butyl-4-carboxy-2-oxo-1-{[3-methyl-2'-(tetrazol-5-yl)-biphenyl-4-yl]-methyl}-1, 2-dihydropyridin

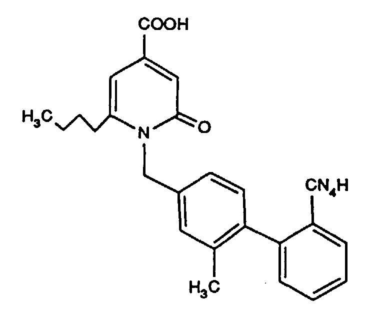

In Analogie zur Vorschrift des Beispiels 43 wird die Titelverbindung aus 475 mg (1,04 mmol) der Verbindung aus Beispiel 4 erhalten.
Ausbeute: 386 mg (84 % der Theorie)
R_{f}: 0,17 (Toluol/Essigester/Eisessig = 10:30:1)

### Beispiel 48

### 6-Butyl-4-carboxy-2-oxo-1-{[2-cyano-2'-(tetrazol-5-yl)-biphenyl-4-yl]-methyl}-1,2-dihydropyridin

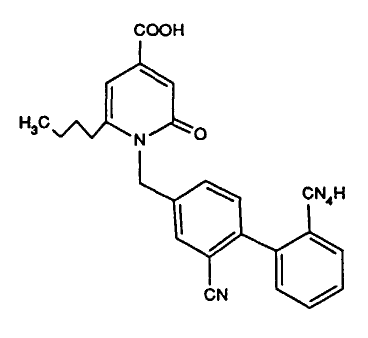

In Analogie zur Vorschrift des Beispiels 43 wird die Titelverbindung aus 234 mg (0,5 mmol) der Verbindung aus Beispiel 5 erhalten.
Ausbeute: 221 mg (97,2 % der Theorie)
R_{f}: 0,22 (Dichlormethan:Methanol:Eisessig 100:10:3)

### Beispiel 49

### 6-Butyl-4-carboxy-2-oxo-1-{[3-methoxycarbonyl-2'-(tetrazol-5-yl)-biphenyl-4-yl]-methyl}-1,2-dihydropyridin

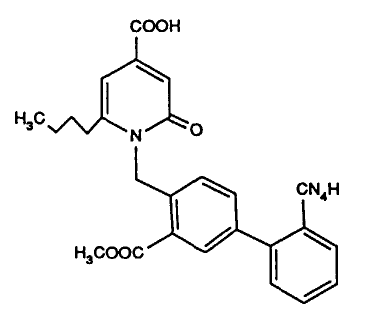

In Analogie zur Vorschrift des Beispiels 43 wird die Titelverbindung aus 89 mg (0,18 mmol) der Verbindung aus Beispiel 31 erhalten.
Ausbeute: 57 mg (66 % der Theorie)
R_{f}: 0,26 (Dichlormethan:Methanol:Eisessig 100:10:3)

### Beispiel 50

### 6-Butyl-4-carboxy-2-oxo-1-{[3-cyano-2'-(tetrazol-5-yl)-biphenyl-4-yl]-methyl}-1,2-dihydropyridin

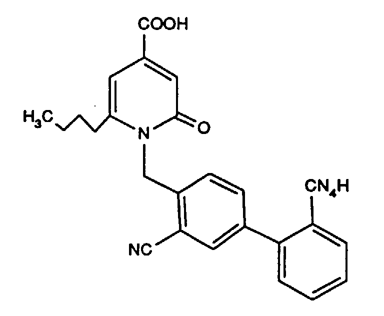

In Analogie zur Vorschrift des Beispiels 43 wird die Titelverbindung aus 1,5 mg (3,2 mmol) der Verbindung aus Beispiel 32 erhalten.
Ausbeute: 1,4 mg (100 % der Theorie)
R_{f}: 0,23 (Dichlormethan:Methanol:Eisessig 100:10:3)

### Beispiel 51

### 6-Butyl-4-carboxy-2-oxo-1-{[3-aminocarbonyl-2'-(tetrazol-5-yl)-biphenyl-4-yl]-methyl}-1,2-dihydropyridin-dinatriumsalz

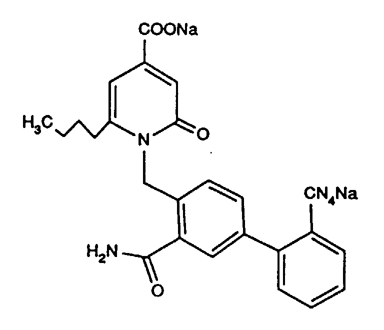

Eine Lösung von 134 mg (0,28 mmol) der Verbindung aus Beispiel 52 und 541 µl (0,54 mmol) einer 1 N Natronlaugelösung in 4 ml Tetrahydrofuran wird 1 h bei 20°C gerührt, mit 10 ml Wasser verdünnt, eingeengt und die verbleibende wässrige Lösung gefriergetrocknet.
Ausbeute: 142 mg (99,6 % derTheorie)
MS(FAB) 495 (M + Na), 517 (M + 2 Na⁻H) 539 (M + 3 Na -2H)

### Beispiel 52

### 6-Butyl-4-methoxycarbonyl-2-oxo-1-{[3-aminocarbonyl-2'-(tetrazol-5-yl)-biphenyl-4-yl]-methyl}-1,2-dihydropyridin

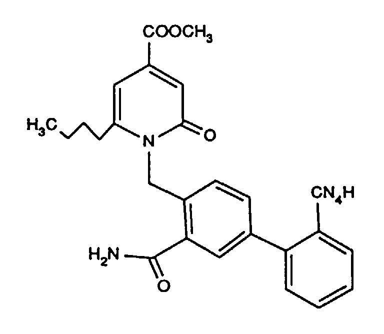

Eine Lösung von 937 mg (2 mmol) der Verbindung aus Beispiel 32 in 20 ml Methanol wird mit Chlorwasserstoffgas gesättigt und 48 h bei 20°C gerührt. Es wird zur Trockne eingeengt, in 100 ml Wasser aufgenommen, und dreimal mit je 30 ml Essigester gewaschen und die vereinigten organischen Phasen dreimal mit je 30 ml Natriumhydrogencarbonatlösung gewaschen. Die wässrigen Phasen werden auf pH = 1 eingestellt, dreimal mit je 30 ml Essigester gewaschen, die vereinigten organischen Phasen über Natriumsulfat getrocknet, eingeengt und an Kieselgel mit Essigester/Methanol/Wasser-Gemischen (20:1:0→100:20:8) zu 472 mg der Titelverbindung chromatographiert.
Ausbeute: 47 % der Theorie
R_{f}: 0,55 (Dichlormethan:Methanol:Eisessig 100:10:3)

### Beispiel 53

### 6-Butyl-4-carboxy-2-oxo-1-{[3-carboxy-2'-(tetrazol-5-yl)-biphenyl-4-yl]-methyl}-1,2-dihydropyridin

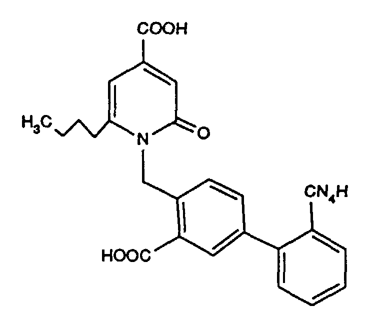

Eine Lösung von 937 mg (2 mmol) der Verbindung aus Beispiel 32 in 4 ml einer 5 N Natronlaugelösung wird 3 h unter Rückfluß erhitzt, bei 20°C mit 30 ml Wasser und 10 ml Essigester verdünnt, auf pH = 1 eingestellt, dreimal mit je 10 ml Essigester gewaschen, die vereinigten organischen Phasen über Natriumsulfat getrocknet, eingeengt und an Kieselgel mit Dichlormethan:Methanol:Eisessig-Gemischen 100:6:1→100:15:5 zu 425 mg der Titelverbindung chromatographiert.
Ausbeute: 44,9 % der Theorie
R_{f}: 0,11 (Dichlormethan:Methanol:Eisessig 100:10:3)

## Patentansprüche

1. Trisubstituierte Biphenyle der allgemeinen Formel I 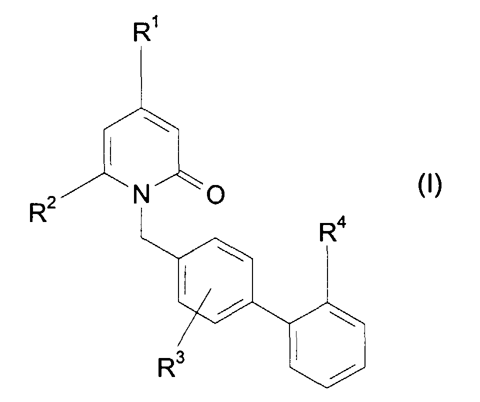 in welcher
R¹ für einen Carboxylrest, oder für einen C₁-C₄-Alkoxycarbonylrest steht,
R² für geradkettiges oder verzweigtes C₁-C₅-Alkyl steht,
R³ für Fluor, Chlor, Hydroxy, Cyano, C₁-C₃-Alkoxy, geradkettiges oder verzweigtes C₁-C₄- Alkyl, Trifluormethyl, Trifluormethoxy, Carboxamido, Carboxy, C₁-C₄-Alkoxycarbonyl oder Nitro steht, und
R⁴ für Tetrazolyl steht,
und deren Salze.

2. Trisubstituierte Biphenyle der Formel nach Anspruch 1, in welcher
R¹ für Carboxy, Methoxycarbonyl oder Ethoxycarbonyl steht,
R² für Propyl, Butyl oder Pentyl steht,
R³ für Fluor, Chlor, Hydroxy, Cyano, Methyl, Methoxy, Ethoxy, Ethyl, Trifluormethyl, Trifluormethoxy, Carboxamido, Carboxy, Methoxycarbonyl, Ethoxycarbonyl oder Nitro steht,
R⁴ für Tetrazolyl steht,
und deren Salze.

3. 6-Butyl-4-methoxycarbonyl-2-oxo-1-[(3-fluor-2'-tetrazol-5-yl-biphenyl-4-yl)-methyl]-1,2-dihydropyridin gemäß Anspruch 1 der Formel 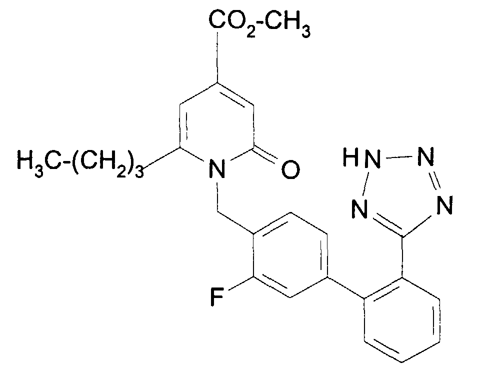 und dessen Salze.

4. Trisubstituierte Biphenyle nach Anspruch 1 bis 3 als Arzneimittel.

5. Verfahren zur Herstellung von trisubstituierten Biphenylen nach Anspruch 1 bis 3 dadurch gekennzeichnet, daß man Pyridone der allgemeinen Formel (II) 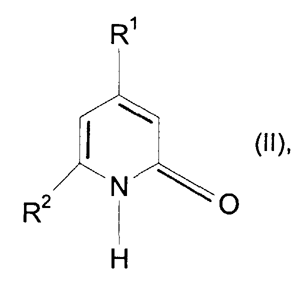 in welcher
R¹ und R² die angegebene Bedeutung haben, mit Verbindungen der allgemeinen Formel (III) 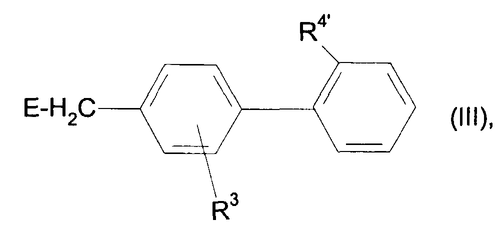 in welcher
R³ die angegebene Bedeutung hat,
R^{4'} für C₁-C₆-Alkoxycarbonyl oder für einen Rest der Formel 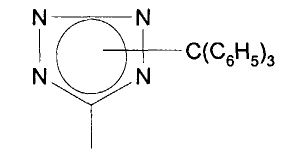 steht, und
E für Chlor oder Brom steht,
in inerten Lösemittel, in Anwesenheit einer Base und gegebenenfalls unter Zusatz eines Katalysators umsetzt.

6. Verfahren zur Herstellung von trisubstituierten Biphenylen nach Anspruch 1 bis 3, dadurch gekennzeichnet, daß man Verbindungen der allgemeinen Formel (IV) 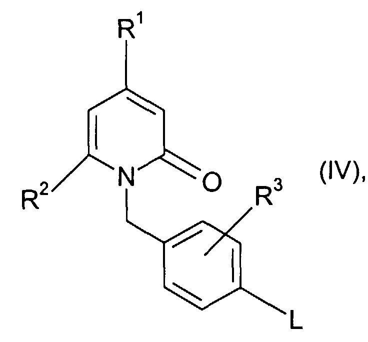 in welcher
R¹, R² und R³ die in Anspruch 1 angegebene Bedeutung haben, und
L für eine typische Abgangsgruppe, wie beispielsweise Brom, Iod, Methan-, Toluol-, Fluor- oder Trifluormethansulfonyloxy, vorzugsweise für Brom steht,
mit Verbindungen der allgemeinen Formel (V) 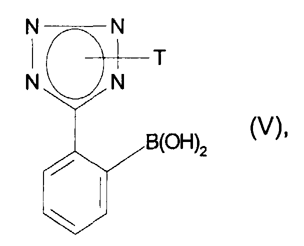 in welcher
T für Wasserstoff oder für die Triphenylmethylgruppe steht,
in inerten Lösemitteln, in Anwesenheit einer Base und metallkatalysiert,
umsetzt,
anschließend die Triphenylmethylgruppe mit Säuren in organischen Lösemitteln und/oder Wasser abspaltet,
im Fall der Garbonsäuren (R⁴/R¹) den entsprechenden Ester verseift und die Verbindungen gegebenenfalls mit Basen in ihre Salze überführt.

7. Arzneimittel enthaltend mindestens ein trisubstituiertes Biphenyl nach Anspruch 1 bis 3.

8. Arzneimittel nach Anspruch 7 zur Behandlung von Bluthochdruck sowie Atherosklerose.

9. Verwendung von trisubstituierten Biphenylen nach Anspruch 1 bis 3 zur Herstellung von Arzneimitteln.

## Claims

1. Trisubstituted biphenyls of the general formula I 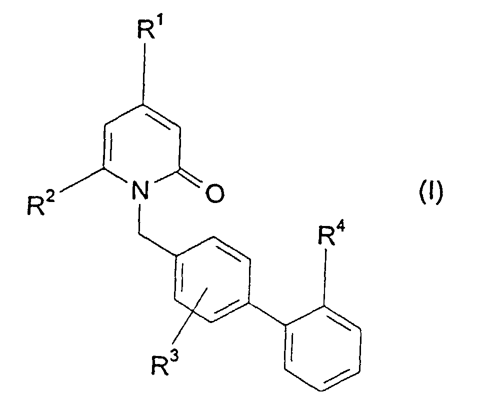 in which
R¹ represents a carboxyl radical or represents a C₁-C₄-alkoxycarbonyl radical,
R² represents straight-chain or branched C₁-C₅-alkyl,
R³ represents fluorine, chlorine, hydroxyl, cyano, C₁-C₃-alkoxy, straight-chain or branched C₁-C₄-alkyl, trifluoromethyl, trifluoromethoxy, carboxamido, carboxyl, C₁-C₄-alkoxycarbonyl or nitro and
R⁴ represents tetrazolyl,
and salts thereof.

2. Trisubstituted biphenyls of the formula according to Claim 1, in which
R¹ represents carboxyl, methoxycarbonyl or ethoxycarbonyl,
R² represents propyl, butyl or pentyl,
R³ represents fluorine, chlorine, hydroxyl, cyano, methyl, methoxy, ethoxy, ethyl, trifluoromethyl, trifluoromethoxy, carboxamido, carboxyl, methoxycarbonyl, ethoxycarbonyl or nitro,
and
R⁴ represents tetrazolyl,
and salts thereof.

3. 6-Butyl-4-methoxycarbonyl-2-oxo-1-[(3-fluoro-2'-tetrazol-5-yl-biphenyl-4-yl)-methyl]-1,2-dihydropyridine according to Claim 1 of the formula 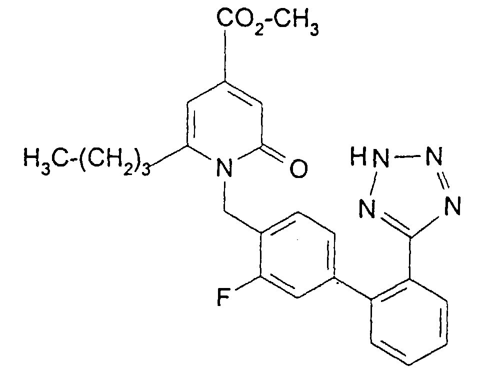 and its salts.

4. Trisubstituted biphenyls according to Claim 1 to 3 as medicaments.

5. Process for the preparation of trisubstituted biphenyls according to Claims 1 to 3, characterized in that pyridones of the general formula (II) 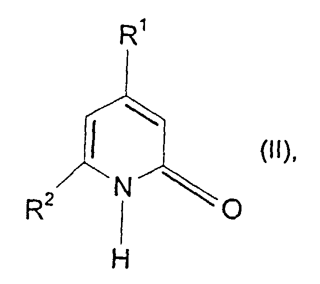 in which
R¹ and R² have the abovementioned meaning, are reacted with compounds of the general formula (III) 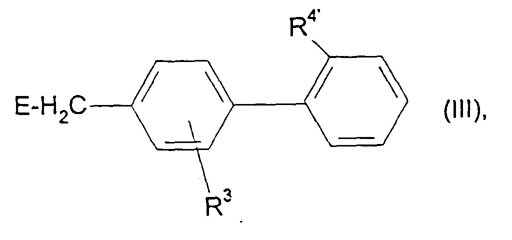 in which
R³ has the abovementioned meaning,
R^{4'} represents C₁-C₆-alkoxycarbonyl or represents a radical of the formula 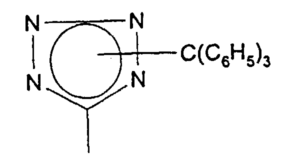 and
E represents chlorine or bromine,
in inert solvents, in the presence of a base and if appropriate with addition of a catalyst.

6. Process for the preparation of trisubstituted biphenyls according to Claims 1 to 3, characterized in that compounds of the general formula (IV) 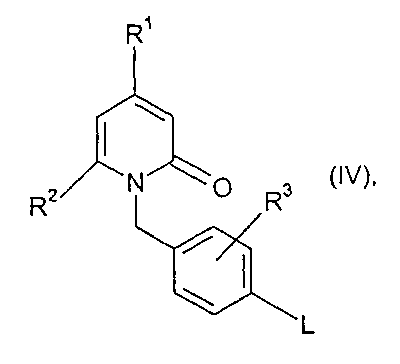 in which
R¹, R² and R³ have the meaning given in Claim 1, and
L represents a typical leaving group, such as, for example, bromine, iodine or methane-, toluene-, fluorine- or trifluoromethanesulphonyloxy, preferably bromine,
are reacted with compounds of the general formula (V) 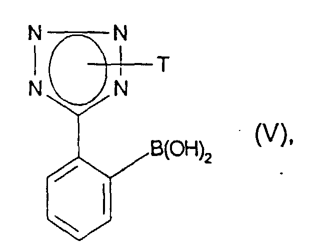 in which
T represents hydrogen, or represents the triphenylmethyl group,
in inert solvents, in the presence of a base and under metal catalysis,
and subsequently the triphenylmethyl group is split off with acids in organic solvents and/or water, and
in the case of the carboxylic acids (R⁴/R¹), the corresponding ester is hydrolysed, and if appropriate the compounds are converted into their salts using bases.

7. Medicament containing at least one trisubstituted biphenyl according to Claim 1 to 3.

8. Medicaments according to Claim 7 for the treatment of hypertension and atherosclerosis.

9. Use of trisubstituted biphenyls according to Claim 1 to 3 for the preparation of medicaments.

## Revendications

1. Biphényles trisubstitués de formule générale I 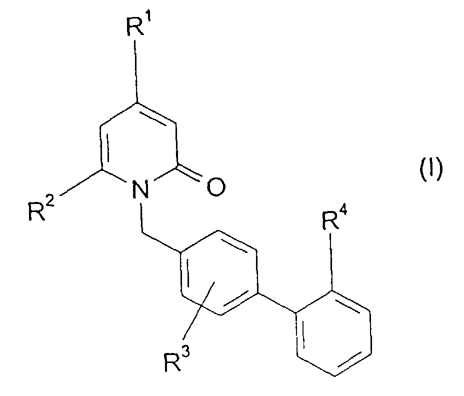 dans laquelle
R¹ est un reste carboxyle ou un reste (alkoxy en C₁ à C₄) carbonyle,
R² est un groupe alkyle linéaire ou ramifié en C₁ à C₅,
R³ représente le fluor, le chlore, un groupe hydroxy, cyano, alkoxy en C₁ à C₃, alkyle linéaire ou ramifié en C₁ à C₄, trifluorométhyle, trifluorométhoxy, carboxamido, carboxy, (alkoxy en C₁ à C₄)carbonyle ou nitro, et
R⁴ est un groupe tétrazolyle,
et leurs sels.

2. Biphényles trisubstitués de formule suivant la revendication 1, dans laquelle
R¹ est un groupe carboxy, méthoxycarbonyle ou éthoxycarbonyle,
R² est un groupe propyle, butyle ou pentyle,
R³ représente le fluor, le chlore, un groupe hydroxy, cyano, méthyle, méthoxy, éthoxy, éthyle, trifluorométhyle, trifluorométhoxy, carboxamido, carboxy, méthoxycarbonyle, éthoxycarbonyle ou nitro,
R⁴ est un groupe tétrazolyle,
et leurs sels.

3. 6-butyl-4-méthoxycarbonyl-2-oxo-1-[(3-fluoro-2'-tétrazole-5-ylbiphényl-4-yl)-méthyl]-1,2-dihydropyridine suivant la revendication 1, de formule 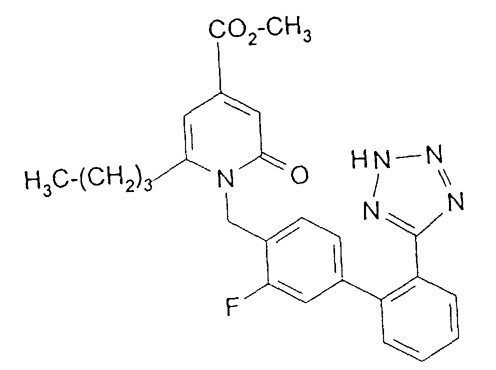 et ses sels.

4. Biphényles trisubstitués suivant les revendications 1 à 3, utilisés comme médicaments.

5. Procédé de production de biphényles trisubstitués suivant les revendications 1 à 3, caractérisé en ce qu'on fait réagir des pyridones de formule générale (II) 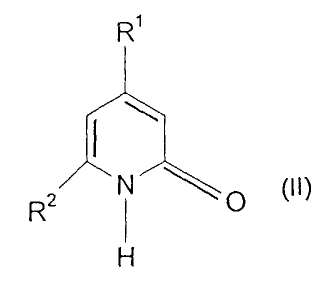 dans laquelle
R¹ et R² ont la définition indiquée ci-dessus, avec des composés de formule générale (III) 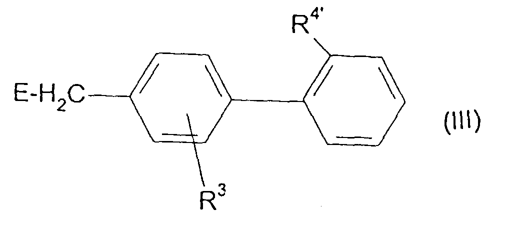 dans laquelle
R³ a la définition indiquée ci-dessus,
R^{4'} représente un groupe (alkoxy en C₁ à C₆)-carbonyle ou un reste de formule 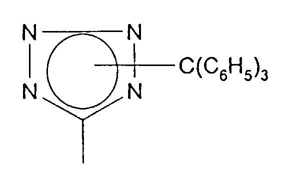 et
E représente le chlore ou le brome,
dans des solvants inertes, en présence d'une base et, le cas échéant avec addition d'un catalyseur.

6. Procédé de production de biphényles trisubstitués suivant les revendications 1 à 3, caractérisé en ce qu'on fait réagir des composés de formule générale (IV) dans laquelle 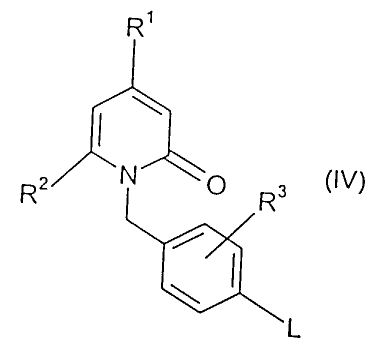
R¹, R² et R³ ont la définition indiquée dans la revendication 1, et
L représente un groupe partant classique, tel que par exemple brome, iode, méthane-, toluène-, fluoro- ou trifluorométhanesulfonyloxy, de préférence bromo,
avec des composés de formule générale (V) 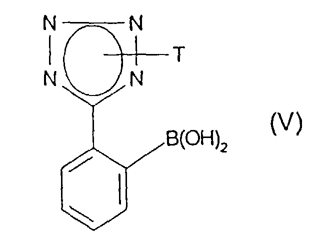 dans laquelle
T représente l'hydrogène ou un groupe triphénylméthyle,
dans des solvants inertes, en présence d'une base et avec catalyse par un métal,
après quoi on élimine le groupe triphénylméthyle avec des acides dans des solvants organiques et/ou dans l'eau,
dans le cas des acides carboxyliques (R⁴/R¹), on saponifie l'ester correspondant et on transforme les composés en leurs sels, le cas échéant avec des bases.

7. Médicament contenant au moins un biphényle trisubstitué suivant les revendications 1 à 3.

8. Médicament suivant la revendication 7, destiné au traitement de l'hypertension ainsi que de l'athérosclérose.

9. Utilisation de biphényles trisubstitués suivant les revendications 1 à 3 pour la préparation de médicaments.
